# EUROPEAN PATENT APPLICATION

(11) **EP 1 918 302 A2**
(43) Date of publication of application: **07.05.2008**
(21) Application number: 07010019.3
(22) Date of filing: 18.05.2007
(51) Int. Cl.: C07K 16/00, C12N 15/00

(54) **Methods for the identification and the isolation of epitope specific antibodies**

(30) Priority: 18.05.2006 US 802085 P
(71) Applicant: Fan, Xiaomin, San Diego CA 92129 (US); Chen, Xiaoming, Savoy, IL 61874 (US)
(72) Inventor: Fan, Xiaomin, San Diego CA 92129 (US); Chen, Xiaoming, Savoy, IL 61874 (US)
(74) Representative: Dendorfer, Claus

(57) **Abstract**

The present invention relates to methods for the identification of an antibody that is specific to an epitope of interest. In certain preferred embodiments, methods of the invention comprise an enrichment procedure and/or a subtractive selection that facilitates the rapid identification of an epitope specific antibody. Methods of the invention also facilitate the identification of an antibody derived from a desired species that is specific for the epitope recognized by another antibody derived from a different species. Methods of the invention further facilitate the identification of an antibody specific to an epitope found in two different antigens, and an antibody capable of binding an epitope of interest but not a homologue of the epitope. Methods of the invention also facilitate the identification of an antibody specific for an antigen presented in one sample, but not, or significantly less, in another sample.

## Description

This application claims the benefit of U.S. Provisional Application No. 60/802,085, filed May 18, 2006; which is incorporated herein by reference.

### 1.0 BACKGROUND OF THE INVENTION

Antibodies are a part of the immune system in many higher organisms. When an organism is invaded by a substance foreign to that organism (also referred to as an antigen), it typically results in the immune system generating antibodies capable of binding the antigen. These antibodies' ability to bind the antigen typically is elevated when compared to their ability to bind molecules other than the antigen, the antibodies thus exhibit binding specificity. Antibodies have been recognized as useful in diagnostics, therapeutics and research, especially in view of their potential to bind a target of interest with high affinity when compared to their binding of other targets.

Antibodies against a target of interest can be generated by immunization methods, whereby the antigen of interest is injected into a host animal, and the antibodies subsequently isolated from the plasma or serum of blood (*see,* for example, Green et al., "Production of Polyclonal Antisera," in Immunochemical Protocols (Manson, ed.), pages 1-5 (Humana Press 1992); Current Protocols in Immunology, John Wiley and Sons, Inc.(2007)). These methods give rise to polyclonal antibodies that comprise a mixture of antibodies that bind the target of interest with different affinities and at different sites or epitopes. Monoclonal antibodies are derived from a single cell clone and represent a homogeneous population of antibodies that typically bind to a more limited part of a target molecule, for example, an epitope. Monoclonal antibodies can be derived from different sources, for example, from mouse, rat, rabbit, or hamster by hybridoma methodologies (*see*, Kohler and Milstein, Nature 256:495-7 (1975)), or they may be obtained through genetic engineering by cloning the antibody genes and expressing them in a bacterial, yeast or mammalian cells (e.g., U.S. Patent No. 4,816,567), or by isolating them from synthetic libraries using methods such as phage or yeast display (McCafferty et al., Nature, 348:552-554 (1990); Clackson et al., Nature 352:624-8 (1991); Marks et al., J Mol Biol.222:581-97 (1991); U.S. Patent No. 5,565,332; Boder and Wittrup, Nat. Biotech. 15:553-7 (1997); U.S. Patent No. 6,699,658; Schreuder et al., Vaccine 14:383-388 (1996); U.S. Patent No. 6,114,147).

Antibodies that can bind an epitope of interest with high affinity, while having a substantially lesser affinity for other epitopes, are of particular interest, for example, in diagnostics, therapy, or research. Moreover, antibodies with a molecular make-up of the organism in which they are used, are highly useful, especially in diagnostic and therapeutic applications.

Hybridoma technology was used to isolate murine, rat or rabbit antibodies with desirable characteristics (high affinity, specificity and/or ligand-blocking activity); however, murine, rat or rabbit monoclonal antibodies have limited therapeutic value in human disease due to the human anti-mouse (HAMA), anti-rat or anti-rabbit response, and the lack of human effector function related to the non-human Fc region (Shawler et al., J Immunol. 135:1530-5 (1985); Hnatowich et al., J Nucl. Med. 26:849-858 (1985); Dillman et al., Cancer Biother. 9:17-28 (1994)). Chimeric antibodies were generated by splicing mouse VH and VL regions onto human heavy chain and light chain constant regions, respectively, followed by expression in a suitable mammalian expression system to help reduce immunogenicity and restore human effector function to the immunoglobulin molecule (Morrison et al., PNAS USA 81:6851-6855 (1984); U.S. Patent No. 5,807,715). Another method for making non-human antibodies more human-like is humanization by CDR (complementarity determining region) grafting, which is based on identifying CDRs by their hypervariable nature (Kabat et al., J. Biol Chem 252:6609-6616 (1977) and Proteins of Immunological Interest, National Institutes of Health, Bethesda MD (1987); www.kabatdatabase.com; IMGT®, the international ImMunoGeneTics information system®, http://imgt.cines.fr) and canonical structure (Chothia and Lesk J. Mol. Biol. 196:901-17 (1987); Chothia et al., J. Mol. Biol. 186:651-63 (1987); Chothia et al., Nature 342:877-83 (1989)) and based on the homology that exists in the framework regions between different families heavy and light chains of mice and humans (*e.g*., reviewed in Pascual and Capra Adv. Immunol. 49:1-74 (1991). Jones et al. (Nature 321:522-525 (1986)) discussed grafting of CDRs from the original mouse monoclonal antibody onto an acceptor human framework accompanied by binding properties reminiscent of the original mouse antibody (e.g., U.S. Patents Nos. 5,225,539; 6,548,640 and 6,982,321). However, CDR grafting typically results in loss of affinity necessitating changes of framework residues back to the original non-human (usually murine) sequence to preserve the correct relative orientation of all six CDRs in the intact immunoglobulin molecule (Verhoyen et al., Science 238:1534-6 (1988); Reichmann et al., Nature 332:323-7 (1988); Tempest et al., Biotechnology 9:266-71 (1991)). Humanization by CDR grafting from the known donor antibody framework to an acceptor human sequence commonly requires computer modeling to guide selection of the acceptor VH and VL genes and the identification of candidate residues that may require back-mutation to the donor sequence to retain binding affinity and specificity. This process often entails iterative testing of antibody variants carrying clusters or the individual mutations predicted to be problematic by computer modeling in order to identify variants that can be tolerated better (Queen et al., Proc. Natl. Acad. Science U.S.A. 86:10029-33 (1989); e.g.,U.S. Patents Nos. 5,585,089 and 6,180,370). While the number of (typically) murine residues in the framework regions can be substantially reduced without loss of affinity, the final humanized antibody constructed by these methods typically retains several murine (or other donor species) framework residues and all murine (or other donor species) CDR sequences.

Obtaining monoclonal antibodies from synthetic VH and VL libraries derived from the desired species is another approach for developing human antibodies (McCafferty et al., Nature, 348:552-554 (1990); Hoogenboom et al., Nucl. Acid Res. 15:4133-7, U.S. Patents Nos. 5,565,332 and 6,544,731; Feldhaus and Siegel, 2004 J. Immunol Meth. 290:69-80; U.S. Patent No. 6,699,658). This approach entails VH and VL libraries from circulating lymphocytes of non-immune (naive) or immune human donors. Such libraries typically comprise unstable VH/VL pairings that are not naturally favored in humans and they are typically built around VH and VL genes of matured antibodies expressed by circulating lymphocytes which carry hypersomatic mutations within the framework regions that may be immunogenic. Not all VH and VL sequences are readily expressed in E.coli, yeast or other non-mammalian hosts.

Transgenic mice carrying human germline VH and VL sequences is an alternative approach that was developed to bypass some of the immunogenicity problems associated with the traditional murine hybridoma technology. However, the process of antibody affinity maturation *in vivo* involves somatic hypermutation, which introduces murine residues into the human frameworks. Therefore, the resultant antibodies could still be immunogenic. Furthermore, identifying the desired antibody from transgenic mice still requires the traditional immunization, hybridoma and screening methods which are labor-intensive.

Isolation of antibodies of interest from a synthetic library can be difficult as requiring considerable screening efforts. Methods for selecting antibodies with antigen-binding properties by ELISA or other solid support-immobilized antigen binding methods have been discussed (*see, e.g.,* Harlow and Lane, Using Antibodies, a Laboratory Manual (1999); Current Protocols in Immunology John Wiley and Sons Inc (2007); U.S. Patent Appl. Publ. No. 2005/0255552). These methods do not identify which of a plurality of antibodies exhibit a desired epitope specificity and desired affinity properties.

In selecting humanized antibodies, original antibody VH and VL sequences may be used to guide selection of human VH and VL elements that can complement the CDRs of the original monoclonal antibody, otherwise known as chain shuffling (also know as guided selection) (*e.g*., Jespers et al., Biotechnology 12:899-903 (1994), Watzka et al., Immunotechnology, 3:279-91 (1998)). Another adaptation for selecting humanized antibodies uses an antibody library from naïve or immune donors constructed by substituting the CDR-3 regions from both VH and VL of the non-human monoclonal antibody for the CDRs in the human antibodies and expression of the library on phage surface for binding to immobilized antigen (U.S. Patent Appl. Publ. No. 2005/0255552). These approaches can lead to loss of potency and epitope drifting relative to the activity of the original non-human monoclonal antibody.

Yeast display of antibody libraries allows for the selection by magnetic cell sorting (MACS) or fluorescence-activated cell sorting (FACS) and others have used antibodies directed against one epitope on an antigen of interest to guide identification of antibodies that bind novel epitopes (Siegel et al., 2004, J. Imunol Meth. 286:141-153; Feldhaus and Siegel, J. Immunol Meth. 290:69-80 (2004)). However, one of the major challenges in antibody engineering/development is the isolation of antibodies that specifically bind to a defined epitope of interest, such as antibodies that bind to a specific site and block the antigen's interaction with its natural ligand. Also, antibody humanization and evolution typically involve the use of methods such chain shuffling or PCR to introduce mutations to either CDR or framework regions (Huse et al., Science 8;246:1275-81 (1989); Marks et al., J Mol Biol.222:581-97 (1991); U.S. Patent No. 5,565,332). These processes often result in a loss of affinity and/or epitope-drifting in the modified antibodies, i.e., the modified antibodies no longer bind to the exact epitope recognized by the parental antibody (e.g., Ohlin et al., Mol Immunol, 33:47-56 (1996); Berry et al., Hybrid Hybridomics, 22:97-108 (2003); Watzka et al., Immunotechnology 3:279-91 (1998)).

Improved methods to identify antibodies and other molecules that can bind epitopes of interest (target epitopes) with high affinity when compared to other epitopes would be highly useful. The current invention provides such methods. The methods of the current invention are particularly well suited for humanization of non-human antibodies with desirable therapeutic or targeting properties. The current invention also provides methods to identify antibodies against potentially useful target antigens where there is no prior knowledge of the exact identity of such antigens.

The current invention further provides methods that enable selection of antibodies directed against an antigen or epitope presented in one sample, but not, or significantly less in the other samples.

### 2.0 SUMMARY OF THE INVENTION

The current invention in certain embodiments relates to methods for the identification of an antibody that is specific for an epitope of interest (epitope specific antibody or target antibody) from a mixture of antibodies. In certain embodiments, methods of the current invention use a marker antibody(s) or ligand (s) to facilitate subtractive selection of antibodies binding to the target antigen. In certain other embodiments, methods of the invention are useful for generating a mixture of antibodies by constructing a synthetic human library, which in certain embodiments contains a single VH and VL gene and is used to identify novel antibodies that block ligand-receptor interactions. In certain other embodiments, a synthetic library is generated from a preferred single VH and VL germline sequence wherein the VH retains the CDRH-3 from the non-human antibody of interest to guide or bias the library towards epitope binders but all other CDRs are selectively randomized at key positions. In certain other embodiments, the methods of the invention are used to humanize a non-human antibody of interest.

In certain embodiments of the invention, a target antibody is identified in a mixture of antibodies by enriching the mixture for the target antibody (enrichment procedure). An enrichment procedure according to certain embodiments comprises removing undesired antibody molecules from the antibody mixture, for example, by exposing the antibody mixture to an antigen that includes the target epitope (target antigen), and then removing antibody molecules that do not bind the target antigen. Complexes comprising the target antigen and an antibody (antigen-antibody complexes) can be separated from other components including antibody molecules, in certain embodiments, by presenting the target antibody attached to a support, for example, a virus, a phage, a cell, E. coli, yeast, a bead, a magnetic bead, a membrane, a matrix. To assist in identifying antigen-antibody complexes, the target antigen is labeled in certain embodiments, for example, with a fluorescent dye.

In certain other embodiments of the invention, the target antibody is further identified and/or isolated by separating it from antibody molecules capable of forming an antigen-antibody complex but that are not specific to the target epitope (non-target antibodies). In certain embodiments of the invention, non-target antibody molecules are separated from target antibodies by a subtractive selection procedure (subtractive selection). A subtractive selection in certain embodiments comprises exposing a target antigen to a mixture comprising target antibody molecules and non-target antibody molecules. In certain preferred embodiments, the subtractive selection procedure further comprises a marker that can bind the target epitope (marker). A marker according to certain preferred embodiments comprises any entity or molecule capable of binding the target epitope. In a subtractive selection, according to certain embodiments, the target antigen is labeled and exposed to a mixture of target and non-target antibody molecules and a labeled marker, with the label of the antigen molecules (antigen label) being distinguishable from the label of the marker (marker label). In certain embodiments, an antibody mixture and a marker are exposed to the target antigen in a subtractive selection under conditions facilitating binding of antibodies and marker to the target antigen. Under these conditions in a subtractive selection, in certain embodiments, binding of a target antibody to the target antigen will inhibit binding of the marker to the target antigen because a target antibody and the marker are both specific for the same site on the antigen, *i.e.,* the target epitope. In certain preferred embodiments, an antibody carrying both antigen label and marker label is a non-target antibody, whereas an antibody carrying only antigen label, and no marker label, is a target antibody. In another certain embodiment, antibody molecules are labeled in a way that is distinguishable from marker label and antigens may remain unlabeled so that in a subtractive selection a target antigen carrying antibody label and no marker label identifies a target antibody.

In another certain embodiment, an antibody is identified that is capable of binding the antigen and the marker, for example, where the antigen and the marker both include the epitope of interest. In certain other embodiments, an antibody is identified that is capable of binding an epitope of interest but not a homologue of the epitope of interest. In certain embodiments, a homologue of an epitope can be the same epitope from a different species. In these embodiments, antibody molecules can be displayed on any support capable of displaying a plurality of antibody molecules of the same epitope specificity, for example, on cells with each cell presenting a plurality of antibody molecules, for example, antibody molecules derived from the same clone. In certain preferred embodiments, a support carrying antigen and marker is identified.

In certain embodiments of the invention, a marker can be used that is a target antibody, for example, an antibody with an undesirable molecular make-up, an antibody from an undesirable species. In certain embodiments of the invention, a marker can be used that is a ligand of some type to the target epitope, for example, a growth factor, a transcription factor, a co-factor, a hormone, a lectin, a peptide.

A method of the current invention, in certain embodiments, can be used to identify an antibody that modulates interactions between entities or molecules, for example, receptor-ligand interactions, protein-protein interactions during complex formation, co-factor-enzyme interactions, protein-DNA interactions, virus-cell surface receptor interactions, transcription factor-transcription factor interactions.

In certain other embodiments, a method of the invention is useful to identify an epitope specific molecule (target molecule) which is capable of specifically binding a target epitope. In certain preferred embodiments, a target molecule may comprise, for example, a protein, a peptide, a small molecule, a glycoprotein.

In certain embodiments, a target antibody identified using a method of the invention can be used for diagnostics or therapeutics, for example, to detect or treat a disease caused by an interaction between entities or molecules wherein the interaction can be modulated by the target antibody.

A method of the current invention, in certain embodiments, can be used to identify antibodies that recognize antigens presented in one sample, but not, or significantly less in a second sample. In certain embodiments, antigens prepared from two different sources are labeled in a way that distinguishes antigens from one sample from another. In certain embodiments, the labeled antigens from different sources are mixed with an antibody mixture. Antibodies are selected that are capable of binding to antigens/epitopes from a first sample, but not, or significantly less, to antigens/epitopes from a second sample. In certain preferred embodiments, antigens may comprise, for example, a protein, a peptide, a glycoprotein, a lipid, a polynucleotide. The current invention, in certain embodiments, provides methods to analyze proteins in samples of interest, such as samples from a diseased and a healthy person or population, for example, with respect to the expression level (abundance) of the proteins and/or with respect to differences in structure or modification between proteins.

Using methods provided in certain embodiments of the current invention, an antibody library, for example, a library comprising millions to billions of different antibodies, can be mixed with antigens from different samples for identification and selection of antibodies specific to the antigens/epitopes present in one sample, but not, or significantly less, in the other samples. These antigen/epitope-specific antibodies, in certain embodiments, can be used to identify the corresponding antigens/epitopes and used to profile protein expression in many different formats including in microarray format.

### 3.0 BRIEF DESCRIPTION OF THE DRAWINGS

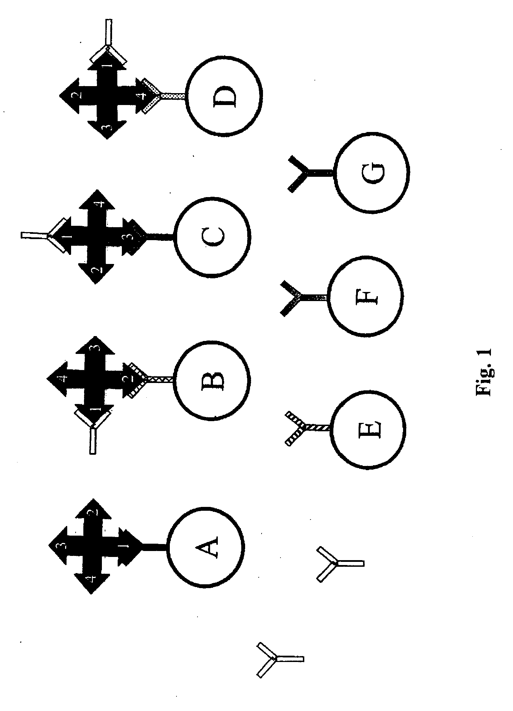

FIGURE 1. Figure 1 shows an example of a subtractive selection for identifying an epitope-specific antibody from a library. The black circles with different letters represent different yeast cells; the different Y shapes on the yeast cells represent different antibodies in the library displayed by different yeast cells; the molecule with numbered arrows represents an antigen of interest that has 4 different epitopes (binding sites); the Y shapes that are not on yeast cells (marker) represent a murine antibody that recognizes the preferred epitope (target epitope), epitope 1, of the antigen.

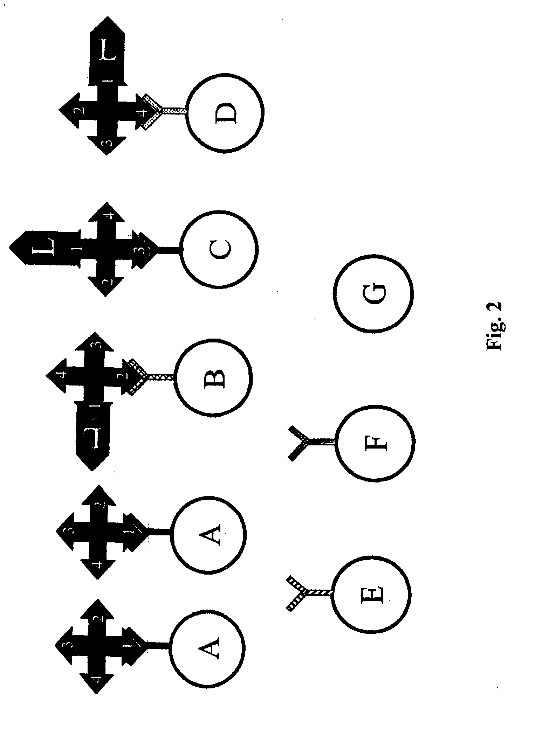

FIGURE 2. Figure 2 shows an example of a subtractive selection for identifying an antibody that inhibits receptor-ligand binding. The ligand of interest (L) (marker) is labeled and is able to interact with the receptor at binding site 1 (target epitope). Yeast cells displaying a library of antibodies are shown as circles with a different letter for each different cell. An antibody displayed by yeast cell A is able to bind to the receptor at binding site 1 (target epitope), therefore its binding to the receptor prevents the binding of the ligand to the receptor. Yeast cells B, C, and D display different antibodies that recognize binding site 2, 3, and 4 (non-target epitopes). The number 1 binding sites ((target epitopes) in these complexes are still available for the ligand binding; therefore, a heterotrimeric complex (yeast cell-antibody-receptor-ligand) is formed that carries both antigen label and marker label (dual labeled).

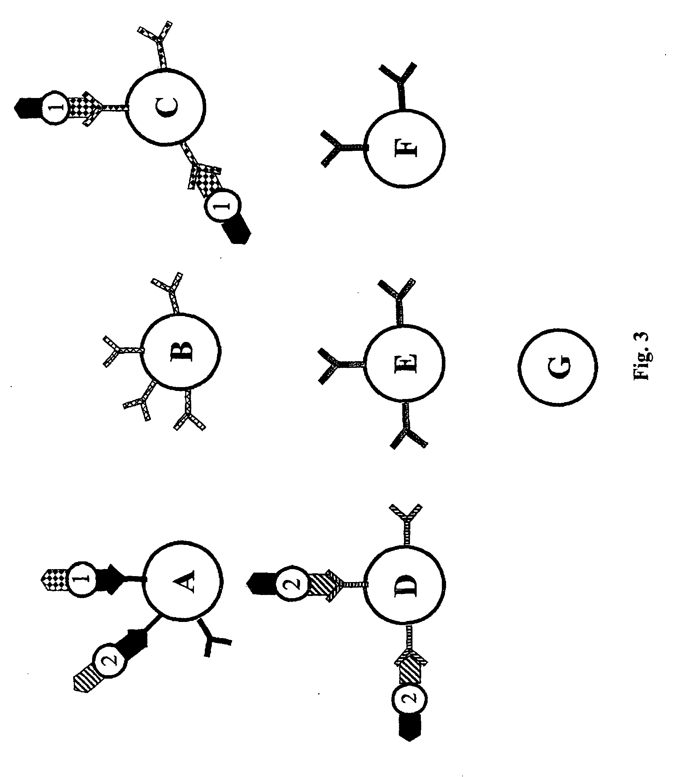

FIGURE 3. Figure 3 shows an example of a subtractive selection for identifying an antibody that recognizes the same epitope shared by two antigens; or antibody specific for one antigen, but not for another homologous antigen. A human antigen of interest (#1) is labeled and is mixed with the rat counterpart of the antigen (#2) that is labeled differently. The labeled antigens are added into a suspension of yeast cells displaying a library of antibodies of any origin. Yeast cell (A) displays an antibody that recognizes the same epitope shared by both the human and rat antigens and yeast cell (A) therefore carries both antigen labels. Yeast cell (C) displaying an antibody that recognizes an epitope specific for the human antigen carries only #1 antigen label. Yeast cell (D) displays an antibody that recognizes an epitope specific for the rat antigen carries only #2 antigen label. Sorting of dual labeled, #1 labeled or #2 labeled only will allow one to select antibodies that recognize both the human and rat antigens, or antibodies that recognize the human antigen specifically (cell C), but not the rat antigen; or antibodies that recognize the rat antigen specifically (cell D), but not the human antigen.

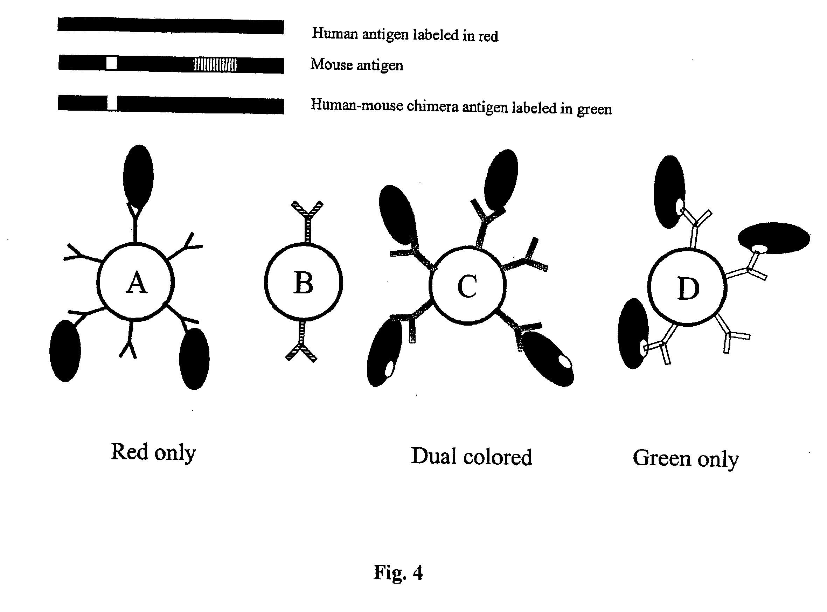

FIGURE 4. Figure 4 shows another example of a subtractive selection for identifying a fully human antibody for an epitope of interest (target epitope). The epitope of interest is grafted from a mouse antigen into the corresponding region of the human counterpart to create a human-mouse chimera antigen. The human antigen is labeled in with a red dye, the human-mouse chimera is labeled in green. Yeast cell (A) displays an antibody that only recognizes the human antigen, but not the human-mouse antigen, so the cell is red only. Yeast cell (C) displays an antibody that recognizes both human and human-mouse chimera, therefore, it is dual-color labeled. Yeast cell (D) displays an antibody that only recognizes the human-mouse antigen, but not the human antigen, therefore, this cell is green only. Antibody displayed by yeast cell (A) only recognizes the epitope that is replaced by the mouse sequence, which is the same epitope recognized by the murine antibody.

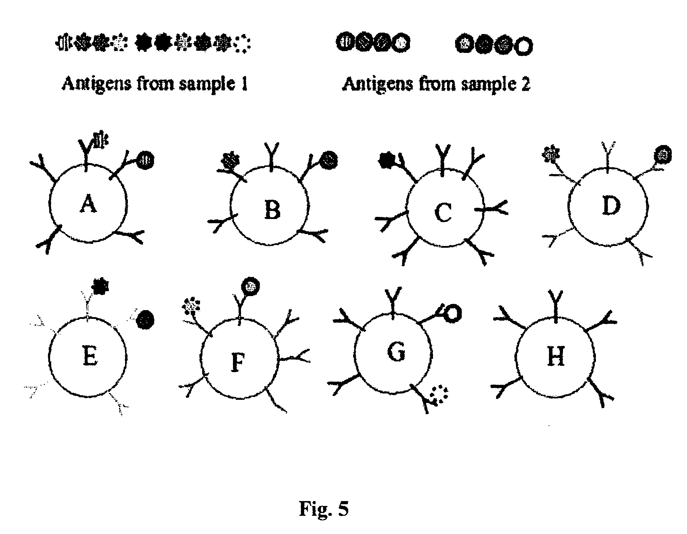

FIGURE 5. Figure 5 shows another example of a subtractive selection for identifying an antibody specific for antigen(s)/epitopes presented in one sample, but not, or significantly less, in another sample. Antigens prepared from two different sources (sample 1 and 2) are labeled in a way that distinguishes the two pools of antigens, such as fluorescent dyes that emits red (represented by broken circle) or green (represented by solid circle). The two pools of labeled antigens are mixed with an antibody library. After incubation in a binding buffer, unbound antigens are washed off with a wash buffer. Cells are subjected to FAC sorting. Cells that emit both red and green lights are removed; cells that emit red only, such as cell C, are selected. Antibodies displayed by cell C recognize antigens/epitopes that are presented in the sample 1, but are absent in the sample 2. Therefore, the selected antibodies (displayed by cell C) are sample 1 specific.

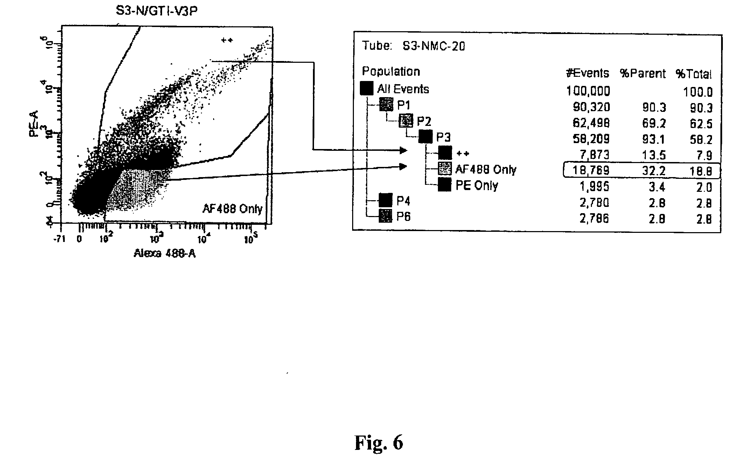

FIGURE 6. Isolation of a humanized antibody from a synthetic library by differential FACS selection. Yeast cells were subjected to two rounds of enrichment for scFv-expressing cells (V5⁺ cells) that bind A1 antigen. In the population used for the S3 sorting step, ~26% of the cells were antigen-positive. An aliquot of the S3 cell population were incubated in A1 antigen, washed and incubated in 100nM GTI-VP3 (a mouse anti-human A1 antibody) and 20 nM NMC-4 chimera followed by Alexa-488-labeled anti-mouse IgG or PE-labeled anti-human Fc antibody. The boxed areas represent the gating boundaries used to define double or single positive cell populations. The double-labeled cell population is indicated by the ⁺⁺ and represent antigen binders that express scFvs that bind to different epitopes to the one recognized by NMC-4. The single-label Alexa-488-positive cells, in contrast, represent antigen binding yeast cells that express scFvs that compete for the NMC-4 binding site.

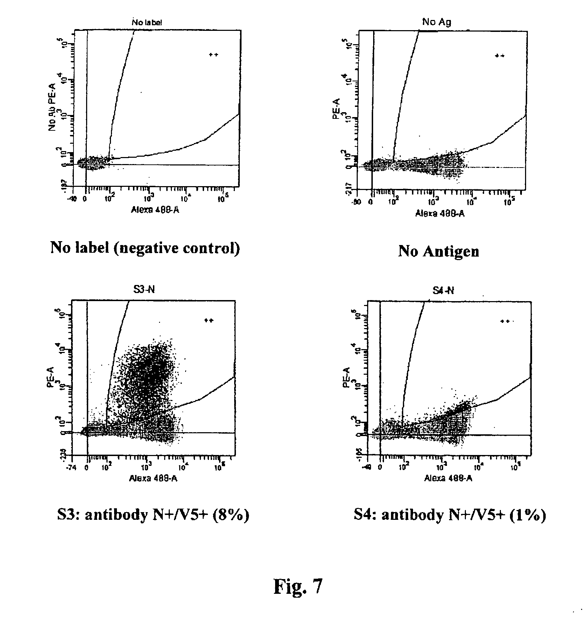

FIGURE 7. Cells were labeled for scFv expression (mouse anti-V5 tag followed by anti-mouse Alexa 488) and their ability to bind NMC-4 chimera (PE-labeled anti-human Fc). The top right hand panel shows the flow cytometry results for the negative control (no fluor labeling), the top right panel shows the results when labeling is performed in the absence of A1 antigen (only V5 tag is labeled to reveal scFv expressing cells). The bottom left panel shows the results of the S3 subtractive selection and the bottom right panel shows the results of the S4 population after the subtractive selection step. As indicated by the gating parameters for the double-positive cell population, these selection steps markedly reduce the number of scFv-expressing cells that are competent to bind NMC-4, which represent epitope-drifted clones.

### 4.0 DETAILED DESCRIPTION OF THE INVENTION

The current invention relates to methods for the identification of an antibody. In certain embodiments, methods according to the invention facilitate the rapid identification of an antibody through a screening method of the invention. In certain preferred embodiments, an antibody that is identified using a method of the invention is capable of binding a target epitope. Another aspect of the invention relates to methods of generating a mixture of antibodies by constructing a synthetic human library, which in certain embodiments contains a single human germline VH and VL gene and is used to identify novel antibodies that block ligand-receptor interactions. In other embodiments, the CDR-H3 of a marker antibody is grafted into the human VH gene. In certain preferred embodiments, synthetic human antibody library is constructed with a single human germline VH that contains the grafted CDRH3 and rationally designed mutations in CDRH1 and CDRH2. In certain preferred embodiments, humanized antibodies that bind to the target epitope recognized by the non-human antibody is isolated using methods of the current invention. Another aspect of the invention relates to methods of isolating antibodies specific for antigen(s)/epitope(s) in one sample, but not, or significantly less in another sample.

### METHODS TO IDENTIFY AN EPITOPE-SPECIFIC ANTIBODY

Methods of the current invention facilitate the identification of an antibody that is specific for an epitope of interest (target epitope) (epitope specific antibody or target antibody). Another aspect of the methods of the invention comprises constructing a synthetic library with a single human germline VH and VL where the CDRs are mutagenized in a manner that the plurality of changes mimics the sequence diversity of the human antibody repertoire.

In certain embodiments, a method of the invention is capable of identifying an epitope specific antibody from a mixture of antibodies that are specific to different epitopes (target antibodies and non-target antibodies). A method of the current invention, in certain embodiments, identifies a target antibody by separating it from non-target antibodies. In certain other embodiments, a method of the invention identifies a target antibody by exposing a mixture of antibodies to an antigen that comprises the epitope of interest (target antigen) and by separating antibodies that are not capable of binding the antigen (enrichment procedure). Antibodies capable of binding the target antigen can be separated from non-binding antibodies, in certain embodiments, by labeling the target antigen in a way that facilitates the separation, for example, through a fluorescent dye, a magnetic bead, or any other means.

In certain other embodiments, a method of the invention identifies a target antibody by exposing a mixture of antibodies to a target antigen in the presence of an entity or molecule that is capable of binding the target epitope (marker) (subtractive selection). In certain preferred embodiments, the antigen molecules in the mixture of antibodies are labeled (antigen label) and the marker is also labeled (marker label), preferably, the antigen label is distinguishable from the marker label. In a subtractive selection according to certain embodiments of the invention, the marker binds to the target epitope unless a target antibody binds to the target epitope. When examining for the presence of labeling in a subtractive selection, in certain embodiments, complexes comprising the target antigen and other components can be detected by examining for antigen label and marker label. In certain preferred embodiments, the presence of antigen label and marker label in a complex suggests the presence of a non-target antibody and the marker in that complex. In certain other preferred embodiments, the presence of antigen label but not the marker label in a complex, suggests the presence of a target antibody in the complex. In certain preferred embodiments, an antibody from a complex comprising antigen label but no marker label is further isolated and characterized. In certain other embodiments, an antibody capable of binding the antigen and the marker are identified, for example, by identifying a support with a plurality of antibody molecules with the same, or substantially the same, epitope specificity that carries antigen label and marker label.

Where a target antigen contains multiple copies of the epitope of interest (target epitope), such as an antigen that forms homo-dimers, -trimers, or -oligomers, a method of the invention can be performed to remove antibodies that bind to different epitopes other than the epitope of interest (target epitope) from the pool of enriched antigen-specific antibodies.

In certain embodiments, after a few rounds of enrichment of antibodies specific for the antigen of interest (target antigen), a marker (marker antibody or ligand) at excess molar ratio to the epitope on the antigen can be incubated with target antigen to occupy all the eptiopes (target epitopes) on the antigen. Upon formation of the antigen-marker complex, the antigen-marker complex can be incubated with a mixture of antibodies. Where all of copies of target epitope have been occupied by the marker (mAb or ligand), antibodies in the antibody mixture (such as a displayed library) that recognize the same epitope of interest (target epitope) are not able to bind the antigen-marker complex. Antibodies that bind to different epitopes (non-target epitope) other than the epitope of interest (target epitope) will form a higher order complex of antibody-antigen-marker. The marker can be labeled in a way to facilitate the removal of antibody-antigen-marker from those antibodies that do not bind maker occupied antigen. Selected antibodies from the antibody mixture (antibody library) therefore, are those that recognize the same binding site (target epitope) on the antigen as does the marker antibody or ligand.

A method of the current invention, in certain embodiments, comprises 1, 2, 3, 4, 5, 6, 7, 8, 9, or 10 rounds of an enrichment procedure. Where a plurality of rounds of an enrichment procedure are applied, one or more rounds of enrichment procedure may differ in their execution from another one or more rounds. A method of the current invention, in certain other embodiments, comprises 1, 2, 3, 4, S, 6, 7, 8, 9, or 10 rounds of a subtractive selection. Where a plurality of rounds of a subtractive selection are applied, one or more rounds of subtractive selection may differ in their execution from another one or more rounds. In certain other embodiments, a method of the current invention comprises an enrichment procedure and a subtractive selection. Certain embodiments of the methods of the invention comprise one or more rounds of an enrichment procedure and one or more rounds of a subtractive selection. In certain embodiments, a method of the invention comprises 1, 2, 3, 4, 5, 1-3, 2-4, or 1-5 rounds of an enrichment procedure and 1, 2, 3, 4, 5, 1-3, 2-4, or 1-5 rounds of a subtractive selection. In certain other embodiments, a method of the invention comprises 1, 2, or 3 rounds of an enrichment procedure, followed by 1, 2, or 3 rounds of a subtractive selection, which may be followed by 1, 2, or 3 rounds of an enrichment procedure, which may be followed by 1, 2, or 3 rounds of a subtractive selection. In certain preferred embodiments, a method of the invention comprises 1 or 2 rounds of an enrichment procedure followed by 1 or 2 rounds of a subtractive selection, which may be followed by 1 or 2 rounds of enrichment procedure.

### ENRICHMENT PROCEDURES

A target antibody or molecule can be identified using a method of the invention, in certain embodiments, by enriching a mixture of antibodies for a target antibody. An antibody mixture contains antibodies specific to a plurality of epitopes and an enrichment procedure, in certain embodiments, increases the fraction of antibody molecules in the mixture that are specific to the epitope of interest (target epitope). In certain preferred embodiments, an enrichment procedure operates by decreasing the number of non-target antibody molecules in the mixture, or by increasing the number of target antibody molecules in the mixture, or by both.

An enrichment procedure, in certain embodiments, separates target and non-target antibody molecules through the target antibodies' ability to bind to an antigen that comprises the epitope of interest (target antigen). Separating target and non-target antibodies can result, in certain embodiments, in an increase in the fraction of target antibodies in the antibody mixture by a percentage that is useful for the identification of target antibodies, for example, an increase of 10 percent or more, 25 percent or more, 50 percent or more, 100 percent or more, 200 percent or more, 300 percent or more, 500 percent or more, 10 to 300 percent, 25 to 200 percent, or 50 to 100 percent. The target antigen can be used to enrich for target antibodies, in certain embodiments, by binding, reversibly or irreversibly, the target antigen or the antibody molecules in the antibody mixture to a solid support. In certain other embodiments, antibody molecules capable of binding the target antigen are isolated by removing unbound antibody molecules or by removing complexes of target antigen and antibody molecules. In certain embodiments, magnetic beads are used as a solid support in combination with a magnet to remove the component carrying the beads through capture by a magnet. In a preferred embodiment, the antigen carries a magnetic bead and an antibody bound to the antigen carrying the magnetic bead will be captured by a magnet. Or, for example, the antigen can be labeled, for example, with a fluorescent dye, and exposed to the antibody. Antigen-antibody complexes may be identified, for example, by FACS, or by binding the antigen molecules to a solid support so that only antibody molecules capable of binding the antigen are retained on the solid support. Variations for enriching target antibodies in an antibody mixture through binding to a target antigen can easily be designed in view of the description provided herein and are within the scope of this invention.

An enrichment procedure, in certain embodiments, comprises exposing an antibody mixture to a target antigen. When the antibody mixture and the target antigen are combined, in certain embodiments, the concentration of both components is preferably such that the number of antibody molecules and antigen molecules is about equal. In certain other embodiments, the number of antigen molecules is higher than the number of antibody molecules, for example, 25 percent higher or up to 25 percent higher, 50 percent higher or up to 50 percent higher, 100 percent higher or up to 100 percent higher, 200 percent higher or up to 200 percent higher, 500 percent higher or up to 500 percent higher, or 1000 percent higher or up to 1000 percent higher. In certain other embodiments, the number of antigen molecules is lower than the number of antibody molecules, for example, 25 percent lower or up to 25 percent lower, 50 percent lower or up to 50 percent lower, 100 percent lower or up to 100 percent lower, 200 percent lower or up to 200 percent lower, 500 percent lower or up to 500 percent lower, or 1000 percent lower or up to 1000 percent lower. The concentration of the antigen relative to the antibody, in certain embodiments, is chosen based on how the binding reaction should be driven. For example, where an antibody mixture likely has a high concentration of antibody molecules that can bind the antigen with high affinity, then it would be desirable to use a lower antigen concentration in the enrichment procedure so that only those antibody molecules are identified that bind the antigen with the highest affinity, and vice versa. Whether an antibody mixture is likely to include a high concentration of antibody molecules that can bind the antigen depends on how related the origins of antigen and antibodies are, how close to a naturally occurring molecule the antigen is, how large the antigen is, and whether the antigen would be expected to evoke a strong immune response.

Exposing an antibody mixture to a target antigen, in certain embodiments, is carried out by combining both in a solvent that facilitates the binding of antibodies to an antigen, for example, a buffer solution, a medium, water, a salt solution, an inorganic solvent, an organic solvent, or any other suitable solvent. When the antibody mixture and antigen are combined in the solvent, the conditions of protein concentration, salt concentration, pH, temperature, and other conditions, are selected to facilitate the binding of an antibody to an antigen, for example, conditions resembling physiological conditions under which antibodies bind an antigen, or any conditions known to facilitate such binding.

A target antigen that is combined with an antibody mixture, in certain preferred embodiments, is labeled or marked in a way that facilitates the identification of binding complexes comprising the antigen and an antibody. Examples of such labeling or marking are a fluorescent dye, a magnetic bead, a tag, an antibody tag, a charge tag or any other label or mark that facilitates the identification of the binding complexes of the antigen and an antibody. In certain embodiments, the complexes of the antigen and an antibody molecule can be separated or enriched by any method capable of recognizing the chosen label, for example, by fluorescence activated cell sorting (FACS) when using a label that is useful for FACS, or by employing a magnet when using a magnetic bead label, or dielectrophoresis when using a charged tag. In certain other embodiments, the antigen is attached to a solid support and the antigen is exposed to the antibody mixture while bound to the support. Preferably, the antigen is removed from the solution in which the antigen is exposed to the antibody mixture so that the antigen and antibody molecules bound to the antigen are separated from antibody molecules that did not bind the target antigen. In certain other embodiments, the target antigen is combined with a mixture of labeled antibodies and target antigen molecules bound to an antibody are further isolated.

### SUBTRACTIVE SELECTION METHODS

A target antibody can be identified, in certain embodiments, through a subtractive selection. A subtractive selection, in certain embodiments, facilitates the identification of a target antibody or an antibody mixture with a high fraction of target antibody molecules. A subtraction selection comprises, in certain embodiments, exposing an antibody mixture to a target antigen and to a molecule capable of selectively binding the target epitope on the target antigen (marker). In certain preferred embodiments, a target antibody in the antibody mixture competes with the marker for binding to the target epitope. In certain other preferred embodiments, a target antibody will bind the target epitope only if its affinity for the target epitope is sufficient to prevent binding of the marker to, or displace bound marker from, the target epitope, and preferably to a degree so that a sufficient number of target antibody molecules bind the target epitope with sufficient strength to facilitate their identification. For example, where the affinity of a target antibody and the marker for the target epitope are about equal, at a given concentration each would bind the equivalent fraction of target epitopes in the subtractive selection, provided no other component would compete for such binding and provided that sufficient numbers of antigen and marker molecules were present. The desired affinity of an identified target antibody when compared to a marker used in the subtractive selection can be scaled, preferably by adjusting the quantity of the marker in the subtractive selection. For example, if one desires to identify a target antibody with higher affinity for the target epitope, a higher amount of marker would preferably be included in the subtractive selection and vice versa. Also, in antibody mixtures, according to certain embodiments, target antibody molecules with different molecular characteristics can be found, for example, target antibody molecules with different affinities for the target epitope and different affinities for a non-target epitope. By using a high concentration of the marker in a subtractive selection, in preferred embodiments, target antibodies with high specificity and high affinity for the target epitope can be identified.

A subtractive selection, in certain embodiments, comprises combining about an equal number of target antigen molecules, marker molecules and antibody molecules in an antibody mixture. When the antigen, marker and antibody mixture are combined, in certain embodiments, the number of antigen and marker molecules separately is higher than the number of antibody molecules, for example, 25 percent higher or up to 25 percent higher, 50 percent higher or up to 50 percent higher, 100 percent higher or up to 100 percent higher, 200 percent higher or up to 200 percent higher, 500 percent higher or up to 500 percent higher, or 1000 percent higher or up to 1000 percent higher. In certain other embodiments, the number of antigen and marker molecules separately is lower than the number of antibody molecules, for example, 25 percent lower or up to 25 percent lower, 50 percent lower or up to 50 percent lower, 100 percent lower or up to 100 percent lower, 200 percent lower or up to 200 percent lower, 500 percent lower or up to 500 percent lower, or 1000 percent lower or up to 1000 percent lower. When the antigen, marker and antibody mixture are combined, in certain other embodiments, the number of antigen molecules is higher than the number of marker molecules, for example, 25 percent higher or up to 25 percent higher, 50 percent higher or up to 50 percent higher, 100 percent higher or up to 100 percent higher, 200 percent higher or up to 200 percent higher, 500 percent higher or up to 500 percent higher, or 1000 percent higher or up to 1000 percent higher. In certain other embodiments, the number of antigen molecules is lower than the number of marker molecules, for example, 25 percent lower or up to 25 percent lower, 50 percent lower or up to 50 percent lower, 100 percent lower or up to 100 percent lower, 200 percent lower or up to 200 percent lower, 500 percent lower or up to 500 percent lower, or 1000 percent lower or up to 1000 percent lower.

A subtractive selection, in certain embodiments, comprises combining labeled antigen in an antibody mixture. When the antigen and antibody mixture are combined, in certain embodiments, the number of antigen is higher than the number of antibody molecules, for example, 25 percent higher or up to 25 percent higher, 50 percent higher or up to 50 percent higher, 100 percent higher or up to 100 percent higher, 200 percent higher or up to 200 percent higher, 500 percent higher or up to 500 percent higher, or 1000 percent higher or up to 1000 percent higher. In certain other embodiments, the number of antigen is lower than the number of antibody molecules, for example, 25 percent lower or up to 25 percent lower, 50 percent lower or up to 50 percent lower, 100 percent lower or up to 100 percent lower, 200 percent lower or up to 200 percent lower, 500 percent lower or up to 500 percent lower, or 1000 percent lower or up to 1000 percent lower. In certain embodiments, after incubation of the labeled antigen with an antibody mixture in a solvent the facilitates antigen-antibody interaction for a period of time, such as 10 minutes, 20 minutes, 30 minutes, 1 hour, 2 hours, 4 hours, up to overnight, unbound antigens are washed off with a wash buffer which can be the same as the binding buffer or another buffer. In certain other embodiments, the bound antigen-antibody complexes are preferably kept on ice to prevent the dissociation of the bound antigen from the antibody. To these antigen-antibody complexes, in certain embodiments, a marker is added. Preferably the marker is labeled and preferably the marker label is distinguishable from a label used on, for example, the antigen. In certain embodiments, the number of marker molecules or entities added is higher than the number of antigen molecules, for example, 25 percent higher or up to 25 percent higher, 50 percent higher or up to 50 percent higher, 100 percent higher or up to 100 percent higher, 200 percent higher or up to 200 percent higher, 500 percent higher or up to 500 percent higher, or 1000 percent higher or up to 1000 percent higher. In certain embodiments, the marker is incubated with the antigen-antibody mixture in a solvent that facilitates marker-antigen interaction for a period of time, for example, for 10 minutes, 20 minutes, 30 minutes, 1 hour, 2 hours, 3 hours, 5 hours, and preferably up to 1 hour on ice. In certain other embodiments, marker molecules that are not bound to an antigen molecule are washed off with a wash buffer which can be the same as the binding buffer or another buffer.

A subtractive selection, in certain embodiments, comprises exposing an antibody mixture to a target antigen and a marker. Exposing an antibody mixture to a target antigen and a marker, in certain embodiments, is carried out by combining those components in a solvent that facilitates the binding of antibodies and of the marker to an antigen, for example, a buffer solution, a medium, water, a salt solution, an inorganic solvent, an organic solvent, or any other solvent for antibody binding assays. Examples of buffers are a PBS, a phosphate buffer, a citrate buffer, a carbonate buffer, or any other buffer suitable for antibody binding assays. Examples of media are any medium useful for growing or maintaining cells, bacteria, viruses, or phages. Examples of a salt solution are a solution comprising any salt suitable for antibody binding assays (e.g., sodium salts, calcium salts, magnesium salts, potassium salts, salts comprising chloride, sulfate, nitrate). When the antibody mixture, the marker, and the antigen are combined in the solvent, the conditions of protein concentration, salt concentration, pH, temperature, and other conditions, are selected to facilitate the binding of an antibody and the marker to an antigen, for example, conditions resembling physiological conditions under which antibodies bind an antigen or, if applicable, conditions under which the marker binds the antigen, or any other conditions known to facilitate such binding.

A target antigen and a marker that are combined with an antibody mixture, in certain preferred embodiments, are labeled or marked in a way that facilitates the identification of binding complexes comprising the antigen and an antibody, and the identification of binding complexes comprising the antigen, an antibody and the marker. The labeling or marking further facilitates, in certain preferred embodiments, distinguishing between antigen-antibody complexes and antigen-antibody-marker complexes. In certain preferred embodiments, two labels are used in a subtraction selection, one preferably for the antigen (antigen label) and another for the marker (marker label). In certain other embodiments, the antibody molecules in the antibody mixture are labeled (antibody label) and the marker is labeled, but not the antigen. Examples of labeling or marking useful for subtraction selection are a fluorescent dye, a magnetic bead, a tag, an antibody tag, a charge tag or any other label or mark that facilitates the identification of the different binding complexes. In certain embodiments, the antigen-antibody complexes that do not include the marker can be separated or enriched by any method capable of recognizing the labels chosen for the subtraction selection, for example, by fluorescence activated cell sorting (FACS), for example, by using two different fluorescent labels useful for FACS and that facilitate distinguishing complexes with and without the marker.

In certain embodiments, a target antibody is identified in a subtraction selection by presenting the antibody mixture bound to a solid support and by identifying antibody-antigen complexes on the support. A support suitable for subtraction selection, in certain embodiments, is a cell, a bacterium, a virus, a phage, a membrane, a magnetic bead, agar, or any other support that facilitate the identification and isolation of a target antibody. In certain other embodiments, the antigen is attached to a solid support and the antigen is exposed to the antibody mixture and the marker while bound to the support. Preferably, the antigen is removed from the solution in which the antigen is exposed to the antibody mixture and the marker so that antibody-antigen complexes are separated from remaining components. In certain other embodiments, the target antigen is combined with a mixture of labeled antibodies and differently labeled marker, and target antigen molecules bound to an antibody, but not the marker, are further isolated.

A subtractive selection, in certain embodiments, facilitates the identification of target antibodies that recognize antigens presented in one sample, but not, or significantly less, in a second sample. A subtraction selection comprises, in certain embodiments, exposing an antibody mixture to antigens from two different sources. The antigens from each source are labeled in a way that antigens from one source are distinguishable from another source. For example, antigens from a first sample are labeled with Alexa 647, which emits a red light under a certain wavelength; while antigens from a second sample are labeled with Alexa 488 (Molecular Probes, OR), which emits a green light and can be detected by most imaging systems. In certain other preferred embodiments, target antibodies will bind the target antigens/epitopes presented in a first sample; such antigens/epitopes are absent or presented significantly less in a second sample. Lack of these antigens/epitopes from a second sample capable of binding to the target antibodies results in the target antibody- displaying cells to emit red light only, or very low green light, if the target antigens/epitopes are presented in a second sample but at very low concentration. The target antibody- displaying cells can be isolated by FAC sorting and expanded for additional rounds of selection. The target antibodies can further be identified by sequencing of the inserts that encode the antibody genes in the isolated cells.

### IDENTIFYING AN EPITOPE SPECIFIC ANTIBODY

In certain embodiments, an antibody specific for an antigen of interest can be identified using the methods of the invention. For example, a molecule (A) that contains an epitope of interest is labeled with a color, such as fluorescent dye that emits a red light. A second molecule (B) that interacts with molecule (A) is labeled with a different color, such as fluorescent dye that emits green light. A library is constructed to display antibodies, proteins, peptides, or small molecules. The vehicle used to display these molecules can be phage, virus, *E. coli*, yeast, cells, beads, or any matrix. A small displaying vehicle can in turn be linked to particles that are suitable for magnetic separation, FACS or dielectrophoretic separation. A yeast cell displayed antibody library will be used as an example in the description below.

Dye labeled (red), or magnetic bead conjugated molecule (A) is incubated with the library to be screened in a binding buffer. After washing off unbound molecule (A), yeast cells displaying antibodies that are able to bind to the labeled molecule A can be selected and enriched from the rest of library by means such as fluorescent-activated cell sorting (FACS), since these yeast cells upon binding to molecule (A) would emit red light under a certain wavelength; or by magnetic bead separation using a magnet. The selected yeast cells can be grown up, induced to express antibodies, and used for the next round of selection.

Magnetic beads separation can be used to quickly separate molecule (A) bound cells from a very large library, so it is preferred method for the first 1-2 rounds selection, if screening of a very large library is required. After the first 1-2 rounds of enrichment, a subtractive selection is incorporated into the selection process. After mixing the labeled molecule (A) with the selected yeast cells, and washing away unbound molecule (A), molecule (B) labeled by a fluorescent dye that emits a different color (i.e. green) is added to the mixture. If an antibody displayed by some of the selected yeast cells binds to the same epitope recognized by molecule (B), molecule (B) will not bind to molecule (A) that already bound to the antibody on the yeast, since the epitope on molecule (A) for molecule (B) is already occupied by the displayed antibody, the resulted yeast cell-antibody-molecule (A) complex should emits red light only.

On the other hand, if an antibody binds to molecule (A) at a different epitope from that recognized by molecule (B), molecule (B) will be able to bind molecule (A), even molecule (A) has formed a complex with the antibody, since the eptiope on molecule (A) for molecule (B) is not occupied by the antibody displayed by the yeast cells. The resulting antibody-molecule (A)-molecule (B) complex will be labeled by dual colors (i.e. green and red). Yeast cells emitting red light only [i.e. yeast cell-antibody-molecule (A)] can be separated from those that are dual colored [i.e. yeast cell-antibody-molecule (A)-molecule (B)] by FACS. Antibody isolated from the yeast cells emitting red light only therefore is able to bind to molecule (A) at the same or overlapping binding site, or epitope, recognized by molecule (B). The isolated antibody can then be used as diagnostic or therapeutic to detect or treat diseases that are caused by the interaction between molecule (A) and molecule (B).

The described method can also be used to select for a fully human antibody that binds to an epitope recognized by an antibody with a different specie origin (i.e, murine antibody) from a fully human antibody library. In this case, the antigen for the murine antibody is molecule (A) and the murine antibody is molecule (B) as described above. One such example is described in more detail below (for example, Examples 1 and 2) and is illustrated in Figure 1.

Antibodies in different forms, such as scFv, Fab, F(ab')2 diabodies can be displayed upon binding to the antigen or molecule (A) as described above, only the binding site on the antigen for the antibody is blocked by the antibody displayed by the yeast cell, the remainder epitopes on the antigen should be exposed. Therefore, in the subtractive selection step, if yeast displayed antibodies bind to epitopes other than that for molecule A, the epitope for molecule (A) on the antibody bound antigen is recognized by molecule (A), and the resulting complexes (yeast cell-antibody-antigen-molecule (A)) can be removed by FACS, since the complexes are dual colored. In this way, antibodies that bind to the eptiope of interest only can be enriched.

In this embodiment of the invention, an antibody library can be generated using synthetic variable regions with randomized amino acid sequences in the CDRs (complementarity-determining region) and human heavy and light chain frameworks, preferably germline frameworks. In another aspect, a fully human antibody library can also be generated by using cDNAs specifically synthesized from human immunoglobulin mRNA isolated from humans, humans immunized with an antigen, or humans with a disease such as infectious, autoimmune, inflammatory or cardiovascular diseases, or cancer. These variable region fragments with diversified CDRs are then cloned into display vectors, such as yeast display vectors. The cloned antibodies are displayed on the surface of yeast cells.

The sequences of the selected human antibodies can be determined by sequencing the variable regions in the display vectors isolated from individual yeast cell. The antibodies can be expressed as soluble form and used in competition assays, such as ELISA (enzyme-linked immunosorbent assay). Since the binding site on the antigen for the selected human antibodies is the same or overlapping with the binding site for the murine antibody, many, if not all of the selected antibodies, are able to compete for binding of the murine antibody in this type of assay. The best human antibody with the highest affinity for the antigen most effectively competes with the murine antibody binding and can be further evaluated for inhibition of the interaction between the antigen and its cognate interacting partner in binding assays.

The fully human antibody finally selected has the same or a very similar binding specificity as the murine antibody, and can be used as a diagnostic or therapeutic antibody. Affinity maturation, if necessary can be performed by generating a subset of variants and selected again with the selection method described above.

### IDENTIFYING AN ANTIBODY THAT INHIBITS MOLECULE-MOLECULE INTERACTIONS

In certain embodiments, an antibody can be identified that can inhibit an interaction between molecules of interest using the methods of the invention. For example, the current invention provides methods for selecting antibodies, proteins, peptides, or small molecules that inhibit protein-protein, ligand-receptor interactions (for example, Examples 3a and 3b and as illustrated in Figure 2). In certain embodiments, such protein-protein interactions include interactions of any proteins found in nature, genetically engineered proteins, animal proteins, plant proteins, primate proteins, cytosolic proteins, nuclear proteins, membrane proteins, receptors, growth factors, enzymes, transcription factors, DNA binding proteins, regulatory proteins, bacterial proteins, viral proteins, soluble proteins.

The method according to certain embodiments of this invention can also be used to isolate an antibody, protein, peptide, or other type of molecule that inhibits protein-protein or receptor-ligand interaction. The schematic shown in Figure 2 represents the case where the method is used to isolate antibodies that inhibit the interaction between a receptor and its cognate ligand. A receptor of interest (X) (represented by the black molecule with 4 arrows representing 4 different epitopes in Figure 2) is labeled in red, the labeled receptor is added to a suspension of yeast cells displaying a library of fully human antibodies. After incubation in binding buffer for 1 hour, unbound receptor is washed away. Labeled yeast cells can be selected and enriched.

After a few rounds of enrichment for yeast cells displaying antibodies that are able to recognize the labeled receptor, labeled receptor is added to the suspension of these yeast cells and incubated for a period of time, such as 1 hour, unbound labeled receptor is washed away with a wash buffer, then excess amount of the cognate ligand (for example, 100 fold higher molar ratio of the ligand over the receptor) labeled in green (represented by the black molecule with a L in it) is added to the yeast cells bound with labeled receptor, and to saturate the binding site still available on the receptor for the ligand, such as the binding site on the receptor bound to yeast cell B, C, D. After incubation for 1 hour, unbound ligand is washed away with a wash buffer.

The washed yeast cells are subjected for FACS sorting. Yeast cells with red only are selected, grown, and used for the next round of sorting. Multiple rounds of sorting can be performed until the majority of yeast cells are red, even in the presence of excess amount of labeled ligand in green. Yeast cells that are the brightest red are selected. The yeast cells finally selected contain vectors encoding antibodies that are able to bind to the receptor of interest in such a manner that blocks the interaction between the receptor and its cognate ligand. The isolated antibodies can be expressed in soluble form and evaluated in receptor-ligand binding assay. The inhibitory activity of these soluble antibodies can be determined in competition binding assay.

Examples 3a and 3b below exemplify the isolation of an antibody that recognizes the receptor and inhibits the receptor's interaction with its cognate ligand using this method.

### IDENTIFYING AN ANTIBODY THAT RECOGNIZES AN EPITOPE FOUND IN ANOTHER PROTEIN

In certain embodiments, the invention also describes methods for selecting antibodies, proteins, peptides, or small molecules that recognize an epitope also found in another protein, for example a homologous protein from a different species, and for selecting antibodies, proteins, peptides, or small molecules that only recognize a molecule from one specie, but not from another, or recognize a particular member molecule in a protein family, but not another member in the same family (for example, Example 4 and as illustrated in Figure 3).

Most antibodies derived from animals are directed against antigens from other hosts and do not recognize antigens from the animals from which the antibodies are produced, in other words, the antibodies do not recognize a self-antigen. The species-specificity of antibodies has been a limitation on the evaluation of antibodies in animals with established disease models. In many cases, surrogate antibodies are used to evaluate the efficacy in the animal models. However, these surrogate antibodies may or may not recognize the same epitope that is recognized by antibodies to be developed as therapeutics. Therefore predicting the actual efficacy for the antibodies in development based on these preclinical data is difficult. Obtaining antibodies that are able to recognize antigens from human and animals with established disease models, therefore, will be highly valuable. The methods of the current invention thus facilitate using the same antibodies in human trials that are evaluated in animal models.

### IDENTIFYING AN ANTIBODY THAT RECOGNIZES A KNOWN EPITOPE IN AN ANTIGEN

The current invention also describes methods for selecting antibodies that recognize a known epitope present in an antigen (for example, Example 5 and as illustrated in Figure 4).

### IDENTIFYING AN ANTIBODY SPECIFIC FOR AN ANTIGEN FOUND IN ONE SAMPLE, BUT NOT, OR SIGNIFICANTLY LESS, IN ANOTHER SAMPLE

In certain embodiments, an antibody specific for an antigen/epitope presented in a first sample, but not, or significantly less in a second sample can be identified using the methods of the invention (for example, as illustrated in Example 6 and in Figure 5). For example, antigens can be prepared from a tumor tissue and a normal tissue as a control. Common abundant molecules, such as albumin, immunoglobulin, transferrin, fibrinogen can be removed using immunodepletion. The remaining low abundant antigens can be enriched.

In certain preferred embodiments, the enriched low abundant antigens from the first sample of interest, such as tumor, can be biotinylated and incubated with a mixture of antibody, such as yeast displayed antibody library. The library used is comprised of different antibodies with members over 10², 10³, 10⁴, 10⁵, 10⁶, 10⁷, 10⁸, 10⁹, 10¹⁰ or larger. Antibodies able to bind the biotinylated antigens are enriched with anti-biotin or streptavidin-conjugated magnetic beads coupling with magnetic beads separation, or with fluorescent-labeled anti-biotin or streptavidin coupling with FAC sorting. The enriched clones can be expanded and used for the next round of selection.

In the next round of selection, in certain preferred embodiments, equal amounts of enriched antigens from the first and second sample can be labeled with different fluorescent dyes, such as Alexa 647 for labeling of the antigens from the first sample; Alexa 488 for labeling of the antigens from the second sample. After labeling, free dyes are removed. Equal amount of labeled antigens from the first and second samples are mixed with a mixture of antibodies displayed on the surface of a support, such as yeast cells in a binding buffer that facilitate the binding of antibodies to the antigens. The antibody-displaying yeast cells can be blocked with unlabeled blocking agents, such as albumin or dry milk, to reduce background binding. The amount of antigens to be mixed with the antibody library can be adjusted so that significant bindings of antigens from the first sample to the antibody library can be readily detected with FACS and the red and green signals are clearly separable.

After the antigens are incubated with the antibody mixture for a period of time, such as 10 minutes, 30 minutes, 1 hour, 2 hours or longer at desired temperatures, such as 4°C, 25°C, or 37°C, unbound antigens are washed off with a wash buffer with or without low concentration of detergent, such as 0.05-1.0 % NP-40 or Triton X-100. The washed cells are subjected to FAC sorting. Cells emitting both red and green lights are removed; while cells emitting red light only are selected. The isolated cells can be expanded and subjected for additional rounds of selection.

In certain preferred embodiments, the selected clones can be induced directly to express antibodies encoded by DNA sequences in these selected cells, or standard molecular biology techniques can be employed to clone the pools of inserts from these selected clones into expression vectors for soluble antibody expression.

In certain preferred embodiments, the expressed antibodies from individual selected clones can be applied/spotted onto a microarray chips using techniques known in the art. The microarry chips can be used to analyze labeled antigens prepared from a pair of samples from different sources as does with antibody microarrays. Antibodies capable of binding antigens prepared from the first sample of interest, such as tumor, from different patients or sources, but that do not bind or weakly bind antigens prepared from the second sample (control), are tumor antigens/epitopes-specific antibodies. These antibodies can be characterized further.

The inserts in the final isolated cells can be sequenced to determine the sequences of the heavy and light chains of the encoded antibody. The isolate antibodies can in turn be cloned into other expression vectors for the production of soluble antibodies in forms of scFv, Fab, F(ab')2, or full length antibodies. These antibodies can be further used to determine their specificity in assays, such as ELISA, Western blotting, or immunohistochemistry staining with samples from which the antigens are derived. Antibodies that recognize antigens from tumor tissues, but not that from normal tissues based on these assay are therefore tumor-specific antibodies.

These tumor-specific antibodies can be further used to isolate and identify the corresponding antigens presented in the tumor tissues. Methods and techniques, such as affinity chromatography and immunoprecipitations, can be used. The identities of the purified antigens can be determined by protein micro sequencing. These identified tumor-specific antigens can be further developed and characterized to serve as biomarkers or targets for therapeutic interventions.

### ANTIBODIES

In certain embodiments, an antibody identified using a method of the invention is antigen specific. An antigen specific antibody, in certain preferred embodiments, is capable of specifically binding the antigen, for example, by binding the antigen with a certain high affinity or higher affinity, or, for example, by not significantly cross-reacting with a epitopes that are related to the antigen of interest. A certain high affinity, in certain embodiments, is a binding affinity (Kₐ) of 10⁵ M⁻¹ or greater, preferably 5 x 10⁵ M⁻¹ or greater, preferably 10⁶ M⁻¹ or greater, preferably 5 x 10⁶ M⁻¹ or greater, preferably 10⁷ M-¹ or greater, preferably 5 x 10⁷ M⁻¹ or greater, more preferably 10⁸ M⁻¹ or greater, preferably 5 x 10⁸ M⁻¹ or greater, preferably 10⁹ M⁻¹ or greater, and preferably 5 x 10⁹ M⁻¹ or greater. The binding affinity of an antibody can be determined through routine analysis, for example, by Scatchard analysis described in Munson et al., Anal Biochem., 107:220 (1980) or by surface plasmon resonance as described, for example by Altschuh et al., Biochemistry 31:6298-6304 (1992). Antibodies from any origin may be identified using a method of the invention, for example, from horse, cow, dog, chicken, rat, mouse, rabbit, guinea pig, goat, or sheep.

A method of the invention can be used to identify a target antibody of any kind, including, but not limited to, polyclonal, monoclonal, multispecific, human, humanized or chimeric antibodies, anti-idiotype antibodies, bispecific, trispecific, or multispecific antibodies, immuno-conjugates, single chain antibodies, Fab fragments, F(ab') fragments, fragments produced by a Fab expression library, F(ab')2 fragments, Fd fragments, single-chain Fvs (scFv), disulfide-linked Fvs (sdFv), fragments comprising either a VL or VH domain, anti-idiotypic (anti-Id) antibodies (including, *e.g.,* anti-Id antibodies to antibodies of the invention), and epitope-binding fragments of any of the above. In certain embodiments, an antibody identified using a method of the invention is an immunoglobulin molecule or an immunologically active portion of an immunoglobulin molecule with an antigen binding site that is capable of specifically binding a suitable antigen. The immunoglobulin molecules of the invention can be of any type (*e.g.,* IgG, IgE, IgM, IgD, IgA and IgY), class (*e.g.,* IgG1, IgG2, gG3, gG4, gA1 and IgA2) or subclass of immunoglobulin molecule.

Methods of the invention are useful for identifying an epitope specific antibody from a mixture of antibodies that includes antibody molecules specific to a plurality of epitopes. A mixture of antibodies, in certain embodiments, may be generated using a method known in the art. For example, polyclonal antibodies may be prepared using methods well-known to those of skill in the art. *See,* for example, Green et al., "Production of Polyclonal Antisera," in Immunochemical Protocols (Manson, ed.), pages 1-5 (Humana Press 1992), and Williams et al., "Expression of foreign proteins in E. coli using plasmid vectors and purification of specific polyclonal antibodies," in DNA Cloning 2: Expression Systems, 2nd Edition, Glover et al. (eds.), page 15 (Oxford University Press 1995). The immunogenicity of an antigen can be increased through the use of an adjuvant, such as alum (aluminum hydroxide) or Freund's complete or incomplete adjuvant.

Or, for example, monoclonal antibodies may be raised as known in the art (*see,* for example, Kohler et al., Nature 256:495 (1975), Coligan et al. (eds.), Current Protocols in Immunology, Vol. 1, pages 2.5.1-2.6.7 (John Wiley & Sons 1991) (Coligan), Picksley et al., "Production of monoclonal antibodies against proteins expressed in E. coli," in DNA Cloning 2: Expression Systems, 2nd Edition, Glover et al. (eds.), page 93 (Oxford University Press 1995)). Monoclonal antibodies can also be obtained from hybridoma cultures by a variety of established techniques. Suitable isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104 (The Humana Press, Inc. 1992)).

Antibody fragments, for example, may be generated, for example, by proteolytic hydrolysis of the antibody. Antibody fragments can be obtained by pepsin or papain digestion of whole antibodies by conventional methods. As an illustration, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, by Goldenberg, U.S. Pat. No. 4,331,647, Nisonoff et al., Arch Biochem. Biophys. 89:230 (1960), Porter, Biochem. J. 73:119 (1959), Edelman et al., in Methods in Enzymology Vol. 1, page 422 (Academic Press 1967), and by Coligan at pages 2.8.1-2.8.10 and 2.10.-2.10.4.

A peptide coding for a single complementarity-determining region (CDR) is another example of an antibody fragment. CDR peptides (minimal recognition units) can be obtained, for example, by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106 (1991), Courtenay-Luck, "Genetic Manipulation of Monoclonal Antibodies," in Monoclonal Antibodies: Production, Engineering and Clinical Application, Ritter et al. (eds.), page 166 (Cambridge University Press 1995), and Ward et al., "Genetic Manipulation and Expression of Antibodies," in Monoclonal Antibodies: Principles and Applications, Birch et al., (eds.), page 137 (Wiley-Liss, Inc. 1995)).

Fv fragments comprise an association of V_{H} and V_{L} chains and may be prepared, for example, as known in the art. These single-chain antigen binding proteins (scFv) can be generated, for example, by constructing a mixture of structural genes comprising DNA sequences encoding the V_{H} and V_{L} domains that may be connected by an oligonucleotide. The structural genes may be inserted into an expression vector, which may be introduced into a host cell, such as E. coli. Preferably, each recombinant host cell synthesizes a single polypeptide chain with a linker peptide bridging the two V domains. Methods for producing scFvs are described, for example, by Whitlow et al., Methods: A Companion to Methods in Enzymology 2:97 (1991) (*also see,* Bird et al., Science 242:423 (1988), Ladner et al., U.S. Pat. No. 4,946,778, Pack et al., Bio/Technology 11:1271 (1993), and Sandhu, *supra*).

Polyclonal anti-idiotype antibodies, for example, can be prepared by immunizing animals with antibodies or antibody fragments specific for an antigen or epitope of interest as known in the art. *See,* for example, Green et al., "Production of Polyclonal Antisera," in Methods In Molecular Biology: Immunochemical Protocols, Manson (ed.), pages 1-12 (Humana Press 1992). *See also,* Coligan at pages 2.4.1-2.4.7. Methods for producing anti-idiotype antibodies are described, for example, by Irie, U.S. Pat. No. 5,208,146, Greene et al., U.S. Pat. No. 5,637,677, and Varthakavi and Minocha, J. Gen. Virol. 77:1875 (1996).

### ANTIBODY LIBRARIES

In certain embodiments, an antibody library is used in a method of the invention. An antibody library is a collection of antibodies, preferably one from which one may identify a target antibody using the disclosed methods. In certain preferred embodiments, an antibody library facilitates the identification of a target antibody in the disclosed methods, for example, by presenting the antibodies on a support. Examples of a support useful for presenting an antibody library are cells, RNA-ribosome, a gel, bacteria, viruses, phages, a filter, a glass plate, glass beads, a matrix, or any other support, or any technology useful to display antibodies and that facilitates presenting antibodies for identification of a target antibody using the disclosed methods.

Examples of libraries include, but are not limited to, antibody libraries constructed using cDNA amplified from mRNA extracted from human spleen or B cells whom have been immunized, non-immunized, or with auto-immune diseases by using immunoglobulin heavy and light chain specific primers. Or, for example, antibody libraries can also be constructed using synthetic heavy and light chains with randomized CDRs. Antibody libraries can be expressed, for example, as scFv, Fab, F(ab')2, diabodies, hAb (*E*.*g*., half antibody, the two cysteines in the hinge region of the heavy chain are mutated so that the heavy chain is not able to pair with another heavy chain to form a full IgG antibody, only one heavy chain and one light chain complex will be displayed. This complex is only half of the full IgG antibody), or full IgG molecules. Or, for example, libraries can also be comprised of cDNAs from cells of any species, such human, mouse, rat, hamster, rabbit or any other species expressing antibodies. Libraries can also be comprised, for example, of random peptides with or without disulfide bond linkage, or of protein fragments, or of small molecules, or of molecules, or combinations of different type of molecules. In certain preferred embodiments, libraries are displayed on the surface of a support, such as virus, phage, E. coli, yeast, mammalian cell, or any type of matrix.

In certain embodiments, an antibody library is expressed in an expression system, including, but not limited to, microorganisms such as bacteria (*e.g*., E. coli, B. subtilis) transformed with recombinant bacteriophage DNA, plasmid DNA or cosmid DNA expression vectors containing antibody coding sequences; yeast (*e.g*., Saccharomyces cerevisiae) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BHK, 293, 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). In certain preferred embodiments, bacterial cells such as Escherichia coli, and more preferably, eukaryotic cells, especially for the expression of whole recombinant antibody molecule, are used for the expression of antibodies. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

Where antibodies are expressed in cells, in certain embodiments, they may be grown as a collection or library of different cell clones. The different clones in the library may be screened for a target antibody using the methods of the invention. Where a clone of interest is identified, the antibodies secreted by the cells may be isolated from the culture medium, ascites fluid, or serum by conventional antibody purification procedures, for example, by affinity chromatography (*e.g*., using protein A or protein G-Sepharose) or ion-exchange chromatography, hydroxylapatite chromatography, gel electrophoresis, dialysis, etc. Also, DNA encoding the antibodies is readily isolated and sequenced using conventional procedures (*e.g*., by using oligonucleotide probes that are capable of binding specifically to genes encoding the heavy and light chains of murine antibodies). The cells expressing antibodies, in certain embodiments, serve as a preferred source of such DNA. Once isolated, the DNA may be placed into expression vectors, which are then transformed into host cells such as E. coli cells, or are transfected into simian COS cells, Chinese Hamster Ovary (CHO) cells, or myeloma cells that do not otherwise produce antibody protein, to obtain the synthesis of antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs., 130:151-188 (1992).

In a further embodiment, antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Synthetic libraries containing only the CDR-L3 and CDR-H3 from the original monoclonal antibody of interest have been constructed as a means to bias the number of epitope binders (also know as guided selection) and aid selection of humanized antibodies of the desired specificity (e.g., Jespers et al., Biotechnology 12:899-903 (1994), Watzka et al., Immunotechnology, 3:279-91 (1998); U.S. Patent Appl. Publ. No. 2005/0255552) but these approaches can lead to loss of potency and epitope drifting.

The DNA that encodes the antibody, in certain embodiments, may be modified to produce chimeric or fusion antibody polypeptides, for example, by substituting human heavy chain and light chain constant domain (C_{H} and C_{L}) sequences for the homologous murine sequences (U.S. Pat. No. 4,816,567; and Morrison et al., Proc. Natl. Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Antibody molecules in an antibody mixture screened using a method of the invention, in certain embodiments, may be attached to a polypeptide tag (or tag) to facilitate identification of a target antibody and to provide sites for attachment of the antibody molecules to a support. In principal, any peptide or protein for which an antibody or other specific binding agent is available can be used as a tag. Affinity tags include a poly-histidine tract, protein A (Nilsson et al., EMBO J. 4:1075 (1985); Nilsson et al., Methods Enzymol. 198:3 (1991)), glutathione S transferase (Smith and Johnson, Gene 67:31 (1988)), Glu-Glu affinity tag (Grussenmeyer et al., Proc. Natl. Acad. Sci. USA 82:7952 (1985)), substance P, FLAG peptide (Hopp et al., Biotechnology 6:1204 (1988)), streptavidin binding peptide, or other antigenic epitope or binding domain. *See,* in general, Ford et al., Protein Expression and Purification 2:95 (1991). DNAs encoding affinity tags are available from commercial suppliers (e.g., Pharmacia Biotech, Piscataway, N.J.). Yeast display of antibody libraries facilitate screening by enabling selection by magnetic cell sortings (MACS) or fluorescence-activated cell sorting (FACS). Antibodies directed against an antigen of interest can be used as a selection marker to guide identification of novel epitopes of interest (Siegel et al., J. Imunol Meth. 286:141-153 (2004); Feldhaus and Siegel, J. Immunol Meth. 290:69-80 (2004)).

Natural human antibodies are derived from 51 different functional VH germlines (Tomlinson et al., Ann NY Acd Sci 764:43-46 (1995); Tomlinson et al., J Mol Biol 256:813-817 (1996); Chothia et al., J Mol Biol 227:799-817 (1992). However, VH germline VH3-23 (DP47) dominates the human antibody repertories (Krikham et al., Embo J 11:603-609 (1992); Brezinschek et al., J Immunol, 155: 190-202 (1995); Kraj et al., J Immunol, 158: 5824-5832 (1997)), indicating that the framework of this germline can support highly diversified CDRs. In addition, VH3-23 pairs with almost all light chains, including VK1, the most common light chain subfamily (de Wildt et al., J Mol Biol 285:895-901 (1999). The combination of VH3-23 and VK1 has been shown to express well and successfully used in the anti-cancer drug Herceptin (Lee et al., J Mol Biol. 340:1073-93(2004). Furthermore, high affinity antibodies have been successfully isolated from phage display libraries constructed with this germline framework (Lee et al., J Mol Biol. 340:1073-93(2004); Pini et al., J Bio Chem 273: 21769-76 (1998); Griffiths et al., Embo J, 13:3245-60 (1994); Sidhu et al., J Mol Biol, 338:299-310 (2004). Therefore, frameworks from germline VH3-23 and VK1 are ideal for construction of human antibody libraries.

The CDR-H3 region of the heavy chain is predominant in antigen recognition and its length varies significantly among different antibodies. For antibody humanization, CDRH3 can be grafted on the VH3-23 germline framework by PCR. For construction of a naive human antibody library, CDRH3 can be generated with degenerate oligos with different lengths encoding rationally designed amino acids with length from 3 up to 20 amino acids. These residues can be randomized with bias, or tailor-randomized, in reflection of the prevalence of different amino acids in natural human antibodies at the corresponding positions. Other CDR residues that are at the periphery of the antigen-binding site and heavily involved in antigen binding, specifically, residues 30-33 of CDR-H1, residues 49, 50 and 52-54 of CDR-H2, residues 28-32 of CDR-L1, residues 50, 53, 55 of CDR-L2, and residues of 91-94 and 96 of CDR-L3, can also be tailor-randomized (Pini et al., J Bio Chem 273: 21769-76 (1998); Hoet et al., Nat Biotechnol 23:344-8 (2005) (Table 2). In addition, residues 56 and 58 of CDR-H2 can also be randomized, since these two residues are highly diversified by somatic mutations in the antibodies derived from *in vivo* immune responses (Sidhu et al., J Mol Biol, 338:299-310 (2004).

When making tailor-randomized oligos, degenerated oligos can be used (Lee et al., J Mol Biol. 340:1073-93 (2004). Alternatively and preferably, trimer codons can be incorporated into the desired position. PCR can be used to link the framework with the designed CDRs.

For antibody humanization, the constructed VH library is comprised of VH3-23 germline framework and CDRH3 from the non-human antibody with CDRH1 and CDRH2 randomized as described above. This VH library is then linked or paired with a library of VL which is constructed to be comprised of a single human germline framework, such as antibody germline from Vk 1 subfamily, and randomized CDRL1-3. VH3-23 and Vk 1 are used as an example. Frameworks from other single germline can also be used wherein the VH3-23 and Vk 1 frameworks are used. Such constructed antibody library will have only CDRH3 from non-human antibody, frameworks from a single VH and VL of human antibody germline, all mutations are within CDRH1-2 and CDRL1-3. The antibodies in such constructed library can be of any kind as discussed in the section entitled Antibodies.

### EPITOPE SPECIFIC MOLECULES AND LIBRARIES THEREOF

In certain embodiments, molecules with epitope specific binding ability, other than antibodies, can also be identified using the methods of the invention. For example, peptides and proteins that are not classified as an antibody or antibody fragment of any kind, but that do exhibit epitope specific binding, can be identified using the methods of the invention. For example, a library of peptides or proteins can be presented in an expression system or display system known in the art or by using a support known in the art, and an epitope specific peptide or protein can be identified using the methods of the invention. Similarly, small molecules that exhibit epitope specific binding can be identified using the methods of the invention. For example, a library of small molecules can be presented in a display system known in the art or by synthesized onto a solid support which will facilitate the separation of epitope-bound molecules from those unable to bind the epitope of interest.

### EPITOPES AND ANTIGENS

Methods of the current invention, in certain embodiments, are useful to identify an antibody or molecule capable of recognizing an epitope of interest or target epitope. An epitope specific antibody can be identified, using the methods of the invention, for any epitope that is capable of specifically binding a suitable antibody. Such epitopes include, but are not limited to, proteins, peptides, nucleic acids, DNA, RNA, glycoproteins, lectins, receptors, viruses, pathogens, hormones, growth factors, growth factor receptors, cytokines, transcription factors, transcription cofactors, steroids, peptide hormones, and any other molecule or atom that has the ability to function as an epitope or antigen.

### MARKERS

In certain embodiments, the methods of the invention use a marker that is capable of binding to the epitope of interest (target epitope). In certain preferred embodiments, the marker is capable of binding the target epitope with sufficient affinity so the marker is useful for the methods of the invention. In certain other embodiments, the lower the affinity of the marker for the target epitope, the more marker molecules may be needed in a subtractive selection according to the invention. Markers useful for the methods of the invention include, but are not limited to, any molecule or atom capable of binding the target epitope, for example, a protein, a peptide, a ligand, a co-factor, DNA, RNA, an antibody, a glycoprotein, a lectin, a receptor, a hormone, a pathogen, a growth factor, a growth factor receptor, a cytokine, a transcription factor, a transcription co-factor, a steroid, an enzyme, small molecules, or any type of molecules of interest that can be labeled with a dye or any type of fluorescence, or can be conjugated to any separable matrix. In certain preferred embodiments, a marker binds only to a single epitope on the antigen used in the methods of the invention, in certain other embodiments, a marker is a small molecule and/or a molecule that is not capable of binding to a large number of antibodies that are specific for distinct epitopes.

### LABELS

In certain embodiments, a label is used in the methods of the invention, for example, to label an antigen and/or to label a marker and/or to label an antibody. In certain embodiments, a label is used in a method of the invention to aid in the identification of a target antibody. A label useful for the invention includes any molecule or atom which can be conjugated, bound, or attached to an antigen, a marker, or an antibody and which can facilitate the identification of a target antibody using the methods of the invention. Examples of such labels include, but are not limited to, a chelator, a photoactive agent, a radioisotope, a fluorescent agent (for example, Alexa-488, FITC, phycoerythrin (PE) Cy 5, Cy 3, Rhodamine or fluorescent quantum dots that can be conjugated onto an antigen, an antibody, or a marker), a paramagnetic ion, or other marker moieties. In certain embodiments, a label can be an antibody conjugated with a label defined above.

### ANTIBODIES IDENTIFIED USING A METHOD OF THE INVENTION AND THEIR USE

Any type of antibody or antibody fragment may be identified using the methods of the invention, as can non-antibody molecules. Antibodies identified with the disclosed methods are useful for diagnostics, therapeutics, or research. Particularly suitable uses for a particular antibody are, for example, the detection of the target epitope, the inhibition of binding reactions involving the target epitope, diagnosis of a condition involving the presence of the target epitope, therapy of a condition requiring the availability of the target epitope, analysis of the binding functions of the target epitope. In many disease conditions, molecular interactions play critical roles for the manifestation of those conditions and detecting the interactions often yields diagnostic information concerning the presence of a condition. Also, modulating those interactions can result in therapeutically desirable changes.

The present invention is further illustrated by the following examples, which are not intended to be limiting in any way whatsoever.

### EXAMPLES

### Example 1: Isolation of a fully human antibody that binds to an epitope recognized by a different antibody, such as a murine antibody.

First, a fully human antibody library is generated using synthetic variable regions with randomized amino acid sequences in the CDRs (complementarity-determining region) and human heavy and light chain frameworks, germline frameworks are preferred. Fully human antibody library can also be generated by using cDNAs specifically synthesized from human immunoglobulin mRNA isolated from humans, humans immunized with an antigen, or humans with certain autoimmune diseases. These variable region fragments with diversified CDRs are then cloned into display vectors, such as yeast display vectors. The cloned antibodies are displayed on the surface of yeast cells.

Antibodies in different forms, such as scFv, Fab, F(ab')2 or hAb can be displayed upon binding to the antigen or molecule (A) as described above, only the binding site on the antigen for the antibody is blocked by the antibody displayed by the yeast cell, the remainder epitopes on the antigen should be well-exposed. Therefore, in the subtractive selection step, if yeast displayed antibodies bind to epitopes other than that for molecule B, the epitope for molecule (B) on the antibody bound antigen is recognized by molecule (B), and the resulting complexes (yeast cell-antibody-antigen-molecule (B)) can be removed by FACS, since the complexes are dual colored. In this way, antibodies that bind to the eptiope of interest only can be easily enriched.

Shown in Figure 1, an antigen of interest is labeled with fluorescent dye in red (represented by the black antibody). A murine antibody that recognizes this antigen is labeled with a fluorescent dye in green (represented by the antibody without fill-in). The labeled antigen is incubated with the yeast cells expressing a library of Fab or hAb in a binding buffer, after a period of time, such as 1 hour; unbound antigens are washed away with a wash buffer. Yeast cells displaying antibodies that recognize the antigen form a complex with the labeled antigen and should be red; these yeast cells can be selected, grown up, and re-selected in the next round of selection.

After the first 1-2 rounds of enrichment, the labeled murine antibody (in green) is added to the mixture containing the yeast cell-antigen complexes in a binding buffer and incubated for a period of time, such as 1 hour. The unbound labeled antibodies are washed away with a wash buffer. The washed yeast cells are subjected to FACS sorting. Yeast cells that bind to labeled antigen only are red as shown in Figure 1 (yeast cell A). Yeast cells with bound antigen that in turn bind to the labeled murine antibody are red and green such as yeast cells B, C, and D in Figure 1. FACS can be used to selectively sort out the yeast cells with red only from the pool of other cells. The selected yeast cells (red only) are those displaying fully human antibodies that are able to bind to the antigen in such a manner that prevents the binding of the murine antibody to the antigen, indicating that the binding site for the isolated human antibody on the antigen is the same or overlapping for the murine antibody.

After FACS sorting, the selected yeast cells (red only) can be grown up, induced to express the encoded human antibodies, and used for the next round selection as described above. The selection can be performed for multiple rounds until the majority of yeast cells are red only, even in the presence of excess amount of the murine antibody in green. Yeast cells that are detected the brightest red are selected for further analysis.

The sequences of the selected human antibodies can be determined by sequencing the variable regions in the display vectors isolated from individual yeast cell. The antibodies can be expressed as soluble form and used in competition assays, such as ELISA (enzyme-linked immunosorbent assay). Since the binding site on the antigen for the selected human antibodies is the same or overlapping with the binding site for the murine antibody, many, if not all of the selected antibodies, are able to compete for binding of the murine antibody in this type of assay. The best human antibody with the highest affinity for the antigen most effectively competes with the murine antibody binding and can be further evaluated for inhibition of the interaction between the antigen and its cognate interacting partner in binding assays.

The fully human antibody finally selected has the same binding specificity as the murine antibody, and can be used as a diagnostic or therapeutic antibody. Affinity maturation, if necessary can be performed by generating a subset of variants and selected again with the selection method described above. A library is generated to have each of the amino acid residue in the CDR regions represented by all 20 different amino acids while the remainder amino acids in the CDR regions are kept unchanged in the same variant. This library is used for affinity maturation with the labeled antigen. The brightest yeast cells are selected.

### Example 2: Isolation of a humanized antibody that binds to an epitope recognized by a different antibody from a non-human species, such as a murine antibody.

The murine monoclonal NMC-4 antibody binds an epitope within the A1 domain of von Willebrandt factor (vWf) in such a manner as to inhibit the interaction of vWf with its platelet receptor, GPlb-α (Shima et al., J Nara Med Assoc., 36:662 (1985); Fujimura et al., Blood, 77:113-20 (1991); Celikel et al., Blood Cells Mol Dis, 23:123-34 (1997)). Another antibody directed against an epitope on the A1 domain, AJW200 has also been described (U.S. Patent No. 5,916,805). These two antibodies were chosen to demonstrate that the epitope-guided selection method described in broad terms above could readily identify and select for human antibody variants that bind the same epitope as NMC-4 from a diverse yeast library pool.

A modified yeast display expression vector was constructed from pYD 1 (Invitrogen, San Diego CA). In the vector, the expression of scFv is controlled by the Gal1/10 promoter, a yeast signal peptide (leader) for the secretion of scFv precedes the scFv gene, which is fused on its C-terminus to a V5 tag that serves as a detection marker on the expressed protein. Downstream of the V5 tag is the gene for the yeast Aga2 protein, which will form two disulfide bonds with membrane-bound Aga1 for displaying scFv on the yeast cell surface. This is in contrast to the previously reported display vector (Feldhaus et al., Nat Biotechnol, 21:163-70 (2003); Immunol Methods 290:69-80 (2004)), in which Aga2 was placed between the signal peptide and the N-terminus of the displayed protein.

In brief, the pYD1 vector was first digested with BsaXI at position 4769 between the pUC origin and GAL1 promoter. The digested vector was blunt ended with Klenow fragment. A Xho I-EcoRI fragment containing the Bsd gene and the TEF1 promoter was isolated from pTEF1/Bsd vector (Invitrogen) digested with Xho I and EcoRI. This fragment was blunt ended and ligated with the blunt end pYD 1 vector. The ligated product was used to transform E. coli and plated on LB plate containing blasticidin. The recombinant plasmid was isolated from the E. coli and transformed into yeast stain EYB100. The transformed yeast cells were now resistant to blasticidin in the medium. Next, the vector was engineered for N-terminal display of scFvs by first using the following primers to amplify a DNA fragment that contains the GAL1 promoter followed by multiple cloning sites as indicated on the primer (Pme I-R)

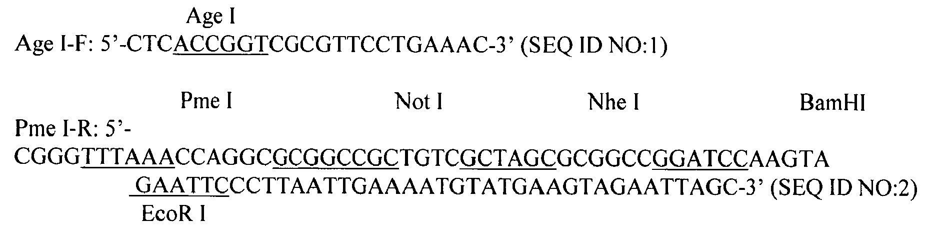

The plasmid pYDI was used as template in this reaction. A PCR product of 524 bp was amplified and digested with Age I and Pme I. The pYD 1 was digested with Age I and Pme I and ligated with the digested PCR product. Recombinant plasmid containing the PCR product was obtained.

The following primers were then used to amplify the aga 2 gene. The pYD 1 vector was used as a template. The PCR product was cloned into the Not I and Pme I site of the above constructed vector. This introduced Not I, Nhe I, and Bam HI cloning sites upstream of the Aga 2 gene. 
Not I
Not-Aga2-F: 5'-GGTAGCGGCCGCCCAGGAACTGACAACTATATGCGAGCAA-3' (SEQ ID
NO:3)
 
Pme I
Pme I-Aga2-R: 5'-CGGGTTTAAACTCAAAAACATACTGTGTGTTTATGGGGCTGC-3' (SEQ
ID NO:4)
 A V5 epitope tag and a (G4S)3 linker was inserted into the vector upstream the aga2. An Xho I site was included upstream of the V5 tag. The entire Gall promoter, leader sequence, V5 tag, and aga 2 fragment was then subcloned into pYD-bsd vector in the Age I and Pem I sites. The final vector was named pYD-N.

An scFv of the murine NMC-4 antibody was designed and custom synthesized based on the based on the published sequence (Celikel et al, Blood Cells Mol Dis, 23:123-34 (1997)). This scFv carried a BamHl site at the 5' end and an XhoI site at the 3' end so it could be readily cloned into the BamHl and Xho1 cloning site of the pYD-N vector. This vector was named pYD-NMC4-scFv. Recombinant plasmid was used to transform the EBY100 yeast strain, which carries a plasmid that expresses Aga 1 (Invitrogen). Individual yeast clones were sequenced to confirm the sequence was correct, and an individual clone was grown in selective medium (7g/L YNB containing ammonium sulfate, 0.8g/L dropout amino acid (-Ura, -Trp), 20g/L glucose, 10.19g/L Na₂HPO₄-7H₂O, and 8.56g/L NaH₂PO₄-H₂). To induce scFv expression, the cell pellet was resuspended in induction medium (20g/L galactose, 20g/L raffinose, and 1g/L glucose) at lx 10⁷ cells/ml and incubated at 20°C for 16-24 hr.

To assess scFv expression levels, the clone was incubated in His-tagged A1 antigen for 1 hour at room temperature followed by incubation on ice for 10 min, then washed and incubated in the presence of Alexa-488-labelled-anti-His tag. The cells were then analyzed by fluorescence microscopy and by flow cytometry. The majority of cells were fluorescent by microscopy and flow cytometry indicated that about 50-70% cells were positive for A1 binding. This confirmed that these cells were effectively displaying NMC-4 scFV.

Comparison of the NMC4 VH region with the germline sequences in the VBASE and Kabat databases database (http://vbase.mrc-cpe.cam.ac.uk/; www.kabatdatabase.com; www.bioinf.org.uk/abs) indicated good homology with the human germline sequence, VH3-23 (DP47). Of the 51 human germline VH genes, this represents a dominant one in the mature human antibody repertoire (Kirkham et al., Embo J, 11:603-9 (1992); Brezinschek et al., J Immunol, 155:190-202 (1995); Kraj et al., J Immunol, 158:5824-32 (1997); IMGT®, the international ImMunoGeneTics® information system http://imgt.cines.fr,) indicating that the framework of this germline can support highly diversified CDRs. In addition, VH3-23 pairs very well with almost all light chains, including VK1, the most common light chain subfamily (de Wildt et al., J Mol Biol, 285:895-901(1999)). Therefore, a synthetic library was constructed whereby only the CDR-H3 of the original 6 murine CDRs was retained in the synthetic VH with rationally designed CDRH1 and 2 and CDRL1-3. Diversity was introduced by tailor-randomizing CDR residues that are at the periphery of the antigen-binding site and heavily involved in antigen binding, specifically, residues 30-33 of CDR-H1, residues 49, 50 and 52-54 of CDR-H2, residues 28-32 of CDR-L1, residues 50, 53, 55 of CDR-L2, and residues of 91-94 and 96 of CDR-L3, were tailor-randomized (Pini et al., J Biol Chem, 273: 21769-76 (1998); Hoet et al., Nat Biotechnol. 23(3): p. 344-8 (2005)) (Tables 1 and 2, below). In addition, residues 56 and 58 of CDR-H2 were randomized, since these two residues are highly diversified by somatic mutations in the antibodies derived from *in vivo* immune responses.

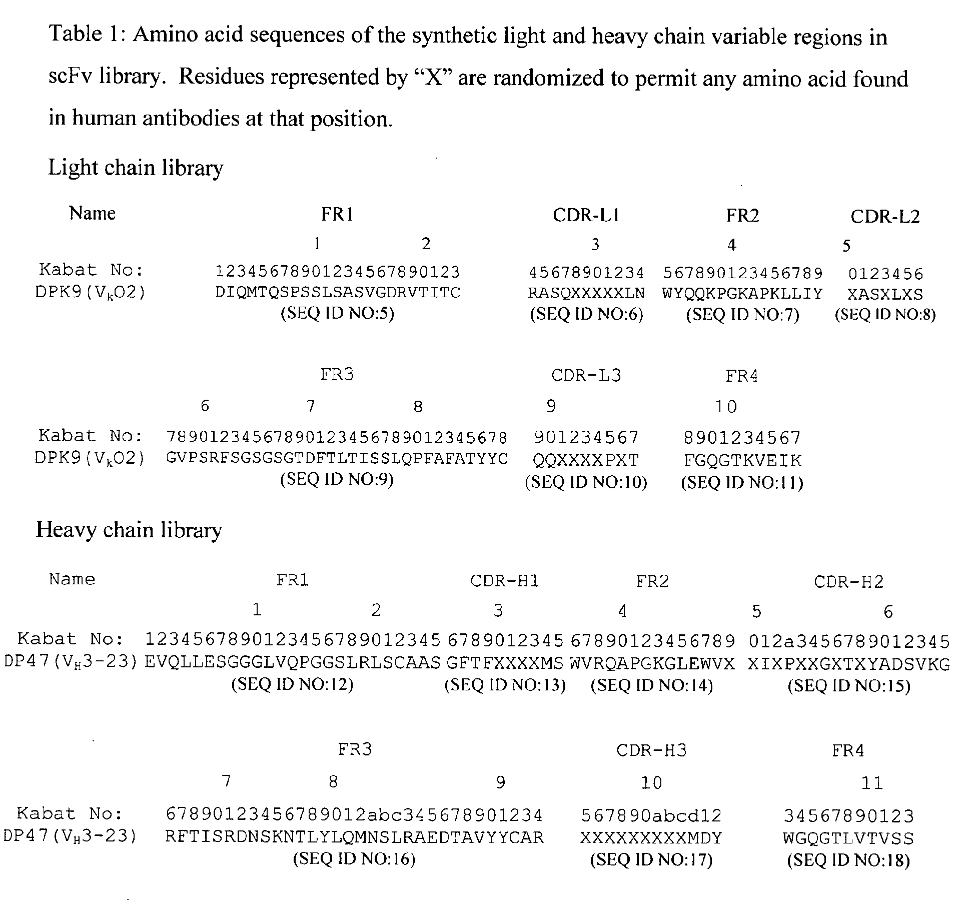

**Table 2: Targeted diversity of CDRs in the synthetic antibody library**

| Positions (VL) | Prevalence in natural antibodies (%) | Positions (VH) | Prevalence in natural antibodies (%) |
|---|---|---|---|
| L1-28 | S(33), N(17), V(17), D(12), G(12), I(3) | H1-30 | S(68), T(18), N(4), R(3), D(2), G(2) |
| L1-29 | 1(40), S(18), V(16), G(12), N(10) | H1-31 | S(50), N(13), G(10), T(10), D(9), R(2), A(1) |
| L1-30 | S(55), N(11), K(11), G(6), R(5), Y(4), T(2), D(2), A(1) | H1-32 | Y(64), S(9), N(7), G(4), F(3), A(3) |
| L1-31 | S(44), N(32), T(11), R(6), I(2), D(2), K(2), G(1) | H1-33 | A(22), Y(20), W(17), G(14), S(12) D(3), T(3), N(2), V(2) |
| L1-32 | Y(67), N(8), W(6), F(5), S(4), D(3), R(2) | H2-50 | R(17), Y(10), W(9), V(9), G(9), I(8), E(8), A(6), S(6), N(6), L(4) |
| L2-50 | G(25), A(22), D(19), W(10), K(8), 1(6), E(3), S(2) | H2-52 | S(26), Y(25), N(17), K(8), I(5), R(3), D(3), T(3) |
| L2-53 | S(36), N(29), T(27), K(3), I(2), R(1) | H2-53 | S(24), D(20), Y(11), G(10), H(9), N(8), I(5), T(3), W(2) |
| L2-55 | A(45), Q(24), E(19), F(3), D(3) | H2-54 | G(37), S(26), D(11), N(7), K(6), F(5), T(4) |
| L3-91 | Y(54), S(12), R(11), A(7), G(4), H(3) | H2-56 | S(28), T(16), N(15), D(10), Y(10), E(5), G(5), A(2) |
| L3-92 | Y(23), G(22), N(15), s(12), D(7), L(6), T(4), H(3), 1(2), | H2-58 | Y(32), N(25), D(12), R(7), S(4),1(4), T(3), H(2) |
| L3-93 | S(46), N(21), Q(7), T(6), H(4), G(3), D(3), R(2) | | |
| L3-94 | S(24), T(23), W(18), Y(11), L(7), F(5), A(3), P(3), V(2), 1 (1) | | |
| L3-96 | L(22), Y(13), W(11), F(9), I (7), R(7), P(3) | | |

A scFv library based on the human germline VH23 and Vk 02 was constructed as follows. First, a synthetic scFv gene encoding VH23, a (GGGGS)4 linker and VkO2 was designed with appropriate restriction sites at the 5' and 3' termini, custom synthesized and cloned into the BamH1 and Xho1 sites of the pYD-V5-Aga2 vector. The integrity confirmed of the final vector was confirmed by sequencing. This plasmid, named pYD-VH23Vk02-N was used as the template for most PCR reactions.

Oligonucleotides were synthesized using tri-nucleotide phosphoamidites (Glen Research, VA) that would elongate the oligos by 3 pre-linked bases at each step. The percentages of each trimer codons at the tailor-randomized positions (*see,* Tables 1 and 2), were based on the prevalence of each amino acid in 3,600 natural human antibodies included in the Kabat database (Kabat et al., "Sequences of Proteins of Immunological Interest," National Institutes of Health, Bethesda MD, (1991); www.kabatdatabase.com; Lee et al., J Mol Biol. 340:1073-93 (2004); Johnson et al., Nucleic Acids Res. 28:214-8 (2000)). Two oligos, CDR3-L-F and CDR1-H-F, with the sequences shown in the Table 4, yielded good quality product but the other three trimer oligos, CDR1-L-F, CDR2-L-F and CDR2-H-F were found to be of poor quality when they were amplified, cloned and analyzed by sequencing. Therefore, these three oligos were replaced with degenerate oligos whose sequences are shown in Table 4 in the construction of the final library.

DNA fragments were PCR amplified with the primers and templates listed in Table 3. The sequences for all these primers are listed in Table 4. To insert the CDRH3 of NMC into the library, the following primers were used in a PCR reaction to amplify the CDRH3: CDR3-F: 5'-GACACCGCTGTTTACTACTGCGCTCGC-3' (SEQ ID NO:19) CDR3-R: 5'-GTACCCTGACCCCAGTAGTCCAT-3' (SEQ ID NO:20)

This 88 bp fragment encoding the NMC-4 CDRH3 was linked with the heavy chain library in the 4th round of PCR. The resultant VH library was linked to a VL library generated with PCR and primers listed in Table 4.

**Table 3: PCR reactions performed to generate the synthetic scFv library with frameworks from human germlines VH3-23 and V_{K}O2, CDRH3 from NMC-4, and rationally randomized CDRH 1-2 and CDRL 1-3**

| DNA fragment | Primers | Template |
|---|---|---|
| 1^{st} round | | |
| VH | | |
| FR1-VH | PYD1-F and HC-R1 | pYD-VH23VkO2-N |
| CDR-H1 | CDR1-H-F2 and CDR1-H-F-R | CDR1-H-F |
| CDR-H2 | CDR2-H-F-F and CDR2-H-F-R | CDR2-H-F |
| FR3-VH | FW3 and HC-R3 | pYD-VH23VkO2-N |
| FR4-VH--linker | FW4-H-F and scFv-linker-R | FW4-H-F and scFv-linker-R |
| VL | | |
| FR1-VL | LC1-F and LC-R1 | pYD-VH23Vk02-N |
| CDR-L1 | CDR1-L-F2 and CDRI-L-F-R-new | CDR-L1 |
| CDR-L2 | CDR2-L-F2 and CDR2-L-R(1-3) | CDR-L2 |
| FR3-VL | FW7-L-F and LC-R3 | pYD- VH23Vk02-N |
| CDR-L3 | CDR3-L-F and CDR3-L-R | CDR-L3 |

| 2^{nd} round | | |
|---|---|---|
| VH Library | | |
| FR1-CDR-H1 | PYD1-F and CDR1-2-H-linker-R | FRI -VH and CDR-H1 |
| CDR-H2-FR3-VH | CDR2-H-F-F and HC-R3 | CDR-H2 and FR3-VH |
| | NMC-CDR3-ATG-F and NMC-CDR3- | |
| NMC-CDR3 | ATG-R | PYD-NMC-scFv |
| VL Library | | |
| FR4-VH-FR1-VL | FW4-H-F and LC-R1 | FR1-VL and CDR-L1 |
| CDR1-2-L-linker - | | |
| CDR-L2 | CDR1-2-L-linker-F and CDR2-L-R(1-3) | CDR-L2 |
| FR3-CDR-L3 | FW7-L-F and FW4-L-R-Xhol | FR3-VL and CDR-L3 |

| 3^{rd} round | | |
|---|---|---|
| VH | | |
| FR1-CDR1-2-FR3- | | |
| VH | PYD1-F and HC-R3 | FR1-CDR-H1 and CDR-H2-FR |
| VL | | |
| | | FR4-VH-FR1-VL and CDR1-2 |
| FR4-VH-CDR-L(1-2) | FW4-H-F and CDR2-L-R(1-3) | CDR-L2 |

| 4^{th} round | | |
|---|---|---|
| VH | | |
| VH-CDRI-2-NMC- | | |
| CDR3 | PYD1-F and NMC-CDR3-ATG-R | FR1-CDRI-2-FR3-VH and NM |
| VL | | |
| VL-library | FW4-H-F and FW4-L-R-Xhol | FR4-VH-CDR-L(1-2) and FR3. |

| 5^{th} round | | |
|---|---|---|
| NMC-scFv-library | PYD1-F and FW4-L-R-Xhol | VH-CDRI-2-NMC-CDR3 and |

**Table 4: List of primers used in the PCR for scFv library generation**

| Primer Name | Sequence |
|---|---|
| pYDl-F | 5' -AAGATGCAGTTACTTCGCTGTT-3' (SEQ ID NO:21) |
| HC-R1 | 5' -AACGGTGAAACCGGAAGC-3' (SEQ ID NO:22) |
| CDR1-H-F2 | 5'-GCTGCTTCCGGTTTCACCGTT-3' (SEQ ID NO:23) |
| CDR1-H-F-R | 5' -CTGACGAACCCAGGACAT-3' (SEQ ID NO:24) |
| CDR2-H-F-F | 5' -GGTAAAGGTCTGGAATGGGTT-3' (SEQ ID NO:25) |
| CDR2-H-F-R | 5'-ACCTTTAACGGAGTCAGCGTA-3' (SEQ ID NO:26) |
| FW3-F | 5' -TACGCTGACTCCGTTAAAGGT-3' (SEQ ID NO:27) |
| HC-R3 | 5' -TCTAGCGCAGTAGTAAACAGCGGT-3' (SEQ ID NO:28) |
| FW4-H-F | 5'-TTCGACTACTGGGGTCAGGGTACCCTGGTTACCGTTTCCTCCG-3' (SEQ ID NO:29) |
| scFv-Linker-R scFV-Linker-R | 5'-GGACTGGGTCATCTGGATGTCAGAACCACCGCCACCGGAACCGCCACCACCGGAGCCACCGCCTCC GGAGGAAACGGTAACCAG-3' (SEQ ID NO:30) |
| LC-F1 | 5' -GACATCCAGATGACCCAGTCC-3' (SEQ ID NO:31) |
| LC-R1 | 5' -TTGGGAAGCACGGCAGGTGAT-3' (SEQ ID NO:32) |
| CDR1-L-F2 | 5'-ATCACCTGCCGTGCTTCCCAA-3' (SEQ ID NO:33) |
| CDR1-L-R-new | 5' -TTTACCCGGTTTCTGCTGGTACCAATTCAG-3' (SEQ ID NO:34) |
| CDR2-L-F-F | 5' -AAAGCTCCGAAACTGCTGATTTAC-3' (SEQ ID NO:35) |
| CDR2-L-R1 | 5'-ACGGGACGGAACACCAGAAGCCAG-3' (SEQ ID NO:36) |
| CDR2-L-R2 | 5'-ACGGGACGGAACACCAGACTGCAG-3' (SEQ ID NO:37) |
| CDR2-L-R3 | 5' -ACGGGACGGAACACCAGATTCCAG-3' (SEQ ID NO:38) |
| FW7-L-F: | 5' -TCTGGTGTTCCGTCCCGT-3' (SEQ ID NO:39) |
| LC-R3 | 5' -GTTGGCAGTAGTAGGTAGCGAAGTC-3' (SEQ ID NO:40) |
| CDR3-L-F | 5' -GCTACCTACTACTGCCAACAA-3' (SEQ ID NO:41) |
| CDR3-L-R | 5' -CTTGGTACCTTGACCGAAGGT-3' (SEQ ID NO:42) |
| CDR1-2-H-linker-R | 5'-ACCCATTCCAGACCTTTACCCGGAGCCTGACGAACCCAGGACAT-3' (SEQ ID NO:43) |
| NMC-CDR3-ATG-F | 5' -GACACCGCTGTTTACTACTGCGCTCGC-3' (SEQ ID NO:19) |
| NMC-CDR3-ATG-R | 5' -GTACCCTGACCCCAGTAGTCCAT-3' (SEQ ID NO:20) |
| CDR1-2-L-linker-F: | 5' -CTGAATTGGTACCAGCAGAAACCGGGTAAAGCTCCGAAACTGCTGATTTAC-3' (SEQ ID NO:44) |
| FW4-L-R-XhoI | 5' -GTTAGGGATAGGCTTACCCCTCGAGCCCTTGATCTCAACCTTGGTACCTTGACCGAAGGT-3' (SEQ ID NO:45) |
| 5'-ext-F2 | 5'-CGGTTTGTATTACTTCTTATTCAAATG-3' (SEQ ID NO:46) |
| 3'-ext-R2 | 5' -CTCCTTGCATTGCCTTCCCG-3' (SEQ ID NO:47) |
| 5'-Ext-F3 | 5'-TACCTCTATACTTTAACGTCAAGGAG-3' (SEQ ID NO:48) |
| 3'-Ext-R3 | 5'-GGCCAAAATAGTAGTCGTTGACAAAG-3' (SEQ ID NO:49) |
| 5'-Ext-F: | 5'-CTGCATAACCACTTTAACTAATACT-3' (SEQ ID NO:50) |
| 5'-Ext-R: | 5' -CTGAAGCAATAACAGAAAATATTG-3' (SEQ ID NO:51) |

Proof reading DNA polymerase, Pfx (Invitrogen) was used for all PCR reactions. In each reaction, greater than 100 ng of the initial templates were used to ensure sufficient diversity of the resultant antibody gene products. All PCR products were separated by 2-4% agarose gel electrophoresis and the DNA bands of the correct anticipated length were excised and the DNA fragment isolated using a Qiagen Gel Extraction Kit.

About 200 bp of DNA was linked to the 5' and 3' of the final PCR product for the scFv library to facilitate the digestion of the PCR product by BamHI and Xho I. A total of 1.6 µg of digested and purified DNA fragment was ligated with 4 µg of digested vector, pYD-N at 16°C overnight. The ligated DNA was ethanol precipitated and used to transform DH10B electro-competent cells. A total of 8.4 x 10⁷ clones was obtained. Sequencing analysis of some these clones indicated that except for CDR-H3, the other CDRs were well diversified and reflected the designed amino acid compositions in these positions. Plasmid DNA was prepared from an E. coli culture of this library and used to transform yeast cell EBY100 for scFv display following the LiOAc transformation protocol (Gietz and Woods, Methods Enzymol. 350:87-96 (2002)). A total of 1.02 x 10⁸ yeast clones were obtained.

To induce scFv expression, transformed yeast cells were grown in selective medium (7g/L YNB containing ammonium sulfate, 0.8g/L dropout amino acid (-Ura, -Trp), 20g/L glucose, 10.19g/L Na₂HPO₄-7H₂O, and 8.56g/L NaH₂PO₄-H₂). For induction, cells pellet was resuspended in induction medium (selective medium with the 20g/L glucose substituted with 20g/L galactose, 20g/L raffinose, and 1g/L glucose) at 1 x 10⁷ cells/ml at 20°C for 16-24 hr.

Preliminary analysis of this library by FACS indicated that 0.5% of clones in the library exhibited His-A1-dependent binding activity (mouse-anti His followed by Alexa 488-labeled goat anti mouse-antibody); this number is much higher than the numbers of potential binders in a unselected naïve library, where on average less than 0.04% of clones exhibit binding activity to a given antigen (Lee et al., Biochem Biophys Res Commun. 346: 896-903 (2006)).

1×10⁹ cells were induced for scFv expression at 20°C for 20 hrs with induction medium. 1.5×10⁹ cells were collected, washed with binding buffer and incubated with 1 µM A1 and anti-V5 antibody for 30 min at room temperature and incubated on ice for 10 min, after washing off unbound A1 and anti-V5 antibody, AJW200 at a final concentration of 200 nM was added to the cell suspension and incubated on ice for 30 min. After washing off unbound AJW200, anti-human-PE (ImmunoResearch Inc) (1:100) and anti-mouse-Alexa 488 (Invitrogen) (1:100) was added into the cell suspension and incubated for 20 min. After washing off unbound secondary labeled antibodies, the cells are subjected to FAC sorting using a FACSAria (Becton Dickenson San Diego CA). Cells with single label, such as cells labeled with PE only, or Alexa 488 only were used to set the gate and compensation parameters. The double positive clones were sorted using a rapid sort mode at a rate of 800 million cells per hour. After a second round of rapid sorting for double positive (antigen and scFv expressing cells) the number of antigen binding cells was increased to 14% of the total yeast cell population.

This population of cells was then incubated in the presence of 500 nM of A1 domain, washed and incubated with 50 nM of NMC-4 chimera and 100 nM of GTI-V3P, a mouse anti-A1 antibody which recognizes the A1 domain but binds to an epitope different from that recognized by NMC-4. After washing off GTI-V3P and NMC-4 chimera, cells were incubated with Alexa 488 labeled anti- mouse antibody and PE-anti-human Fc antibody and sorted at a slower (purity mode) rate of 1 million per hour into a double positive fraction and an antigen binding (GTI-V3P positive), but NMC-4 negative fraction (subtraction selection) (Figure 6). About 8% of the S3 were labeled by A1 antigen and NMC-4, this represent 57% of the antibodies enriched (a total of 14% antigen positive clones in the enriched population) bind to epitopes different from the epitope (target epitope) recognized by NMC-4. When a higher concentration of NMC antibody was used in labeling, a higher percentage of cells labeled by A1-NMC-4 labeling was observed, indicating greater than 57% of antibody clones enriched exhibited some degrees of epitope drifting.

The double positive cells were then analyzed by fluorescence microscopy to assess the ability of individual cells to bind antigen and to bind NMC-4 chimera or AJW200 (recognizes the non-desired epitope). Most of the double positive cells were able to bind NMC-4 and thus represented cells expressing scFvs that showed epitope drifting (*i.e.,* the antibodies bind to eptiopes other than the original epitope recognized by NMC-4). In contrast, the cells isolated as single labeled (positive for antigen but negative for NMC-4 binding) by FACS nearly all stained negatively for NMC-4. These results showed that this subtractive selection step led to a substantial enrichment of antibody clones recognizing the same epitope with an acceptable affinity and eliminated antibodies that bind epitopes (non-target epitope) other than the epitope (target epitope) recognized by the original mouse antibody. Following the subtractive selection step, the single-labeled cells isolated from the S3 population was amplified, induced to express antibody and subjected to two additional rounds of sorting to select cells that were positive for antigen binding but negative for NMC-4 binding. 76% of cells within the yeast population bound antigen and of these, only 1% bound NMC-4, showing that this selection step had resulted in substantial enrichment of clones expressing antibodies of the desired epitope specificity. Ten individual clones from this antigen-only labeled population were grown and analyzed in more detail by fluorescence microscopy. One subset was stained for AJW200 binding and another for NMC-4 binding using 200nM concentration of each antibody. All 10 clones stained for AJW200, while eight of 10 were completely negative for NMC-4 staining. The remaining two showed slight fluorescence with NMC-4, suggesting that these two humanized antibody clones bind to epitopes that are slightly different from the target epitope recognized by the mouse NMC-4 antibody.

The fully human antibody finally selected had the same binding specificity as the murine antibody, and can be used as a diagnostic or therapeutic antibody. Affinity maturation, if necessary can be performed by generating a subset of variants and selected again with the selection method described above.

This example illustrates how the current invention provides a method for antibody humanization whereby highly humanized antibodies that bind to the exact same epitope (target epitope) recognized by the non-human antibody can be rapidly selected.

### Example 3a: Isolation of an antibody that inhibits receptor-ligand interaction.

The method described by this invention can also be used to isolate an antibody, protein, peptide, or other type of molecule that inhibits protein-protein or receptor-ligand interaction. In this example, the invented method can be used to isolate antibodies that inhibit the interaction between a receptor and its cognate ligand. Shown in Figure 2, a receptor of interest is labeled in red (represented by the black molecule with 4 arrows representing 4 different epitopes), the labeled receptor is added to a suspension of yeast cells displaying a library of fully human antibodies. After incubation in binding buffer for 1 hour, unbound receptor is washed away. Labeled yeast cells can be selected and enriched.

After a few rounds of enrichment for yeast cells displaying antibodies that are able to recognize the labeled receptor, labeled receptor is added to the suspension of these yeast cells and incubated for a period of time, such as 1 hour, unbound labeled receptor is washed away with a wash buffer, then excess amount of the cognate ligand (for example, 100 fold higher molar ratio of the ligand over the receptor) labeled in green (represented by the black molecule with a L in it) is added to the yeast cells bound with labeled receptor, and to saturate the binding site still available on the receptor for the ligand, such as the binding site on the receptor bound to yeast cell B, C, D. After incubation for 1 hour, unbound ligand is wash away with a wash buffer.

The washed yeast cells are subjected for FACS sorting. Yeast cells with red only are selected, grown, and used for the next round of sorting. Multiple rounds of sorting can be performed until the majority of yeast cells are red, even in the presence of excess amount of labeled ligand in green. Yeast cells that are the brightest red are selected.

The yeast cells finally selected contain vectors encoding antibodies that are able to bind to the receptor of interest in such a manner that blocks the interaction between the receptor and its cognate ligand. The isolated antibodies can be expressed in soluble form and evaluated in receptor-ligand binding assay. The inhibitory activity of these soluble antibodies can be determined in competition binding assay.

Similarly, this approach can be used to isolate antibodies that recognize the ligand and inhibit the ligand's ability to bind its receptor. In this case, the labeled ligand is added to the suspension of yeast cells displaying an antibody library first. After enriching yeast cells displaying antibodies for the ligand, excess amount of labeled receptor is added to the suspension of yeast cell-ligand complexes. For those receptors that are difficult to purify or for which separation of the receptor from cell membrane changes its conformation, labeled cells or labeled membrane preparations bearing the receptor can be used in the place of purified labeled receptor. After incubating and washing, the yeast cells are subjected for FAC sorting.

Only those yeast cells with ligand labeled in green attached are selected. These cells therefore display antibodies that are able to bind the ligand and inhibit its receptor from binding to the ligand. The selected yeast cells are grown and used for the next round of sorting until the majority of the cells are green. Antibodies displayed by yeast cells with the brightest green are selected for further analysis.

Again, these antibodies are expressed as soluble antibodies and evaluated in receptor-ligand binding assay. The isolated fully human antibodies can be used as a diagnostic or therapeutic antibody. Yeast cell A in Figure 2 can be easily isolated from the pool of yeast without labeling or labeled with dual colors. Isolation of yeast cells with red (yeast cell A) only facilitates the isolation of an antibody that recognizes the receptor and inhibits the receptor's interaction with its cognate ligand.

### Example 3b: Isolation of an antibody that inhibits a receptor-ligand interaction.

The method described by this invention can also be used to isolate an antibody, protein, peptide, or other type of molecule that inhibits protein-protein or receptor-ligand interaction. In this example, the invented method can be used to isolate antibodies that inhibit the interaction between a receptor and its cognate ligand as shown in Figure 2. To demonstrate this, a library with randomized CDRs was constructed and then screened to identify scFvs that bound the uPA receptor (uPAR, antigen) in a manner that competed for binding to its ligand, uPA. The library was constructed as described in example 2, with the exception that in this case the CDR-H3 was also completely randomized (i.e., positions 95 through 101 in the V3-23 germline sequence). A library of 5.5×10⁹ clones was generated. Large-scale plasmid was prepared and used to transform yeast stain EYB 100. A total of 1.1 ×10¹⁰ clones was obtained.

The antigen, the uPA receptor (uPAR) and its ligand, uPA were cloned from the IMAGE clones 65768 (ATCC, contains containing human uPAR (1-238 aa) and Clone #3890980 (Invitrogen, containing human uPA (1-135 aa), respectively. uPAR was cloned into the *pcDNA6*/*myc-His C vector* using the following primers
a. uPAR-BamHI-F: 5'-CCGGATCCGCCGCCACCATGGGTCACCCGCCGCTGCT-3' (SEQ ID NO:52)
b. uPAR-His-Agel-R: 5'-ATGACCGGTCCCACTGCGGTACTGGACATC-3' (SEQ ID NO:53)

The PCR product was amplified from the Image clone and digested with BamHI and Age I. The digested product was then ligated with BamHI and Age I digested pcDNA6/myc-His C vector. The PCR product includes DNA sequences encoding the leader sequence ofuPAR for secretion of the expressed uPAR (1-238 aa, lacking the membrane anchor domain). The secreted uPAR has a His tag at its C-terminus (derived from the vector). Five clones with inserts were sequenced and analyzed. Two mutations at amino acid 114 and 263 were found in both the cloned uPAR and the original template compared to the sequence in Genebank, Accession No# NM-002659. The mutations were corrected by standard recombinant PCR techniques.

The following primers were used to sub-clone uPA into the pcDNA6 vector.
c. uPA-BamHI-F: 5'-CGGGATCCGCCGCCACCATGAGAGCCCTG-3' (SEQ ID NO:54)
d. uPA-Xhol-R: 5'-CCGCTCGAGCTTTTCCATCTGCGCAGTCATG-3' (SEQ ID NO:55)

The PCR product was amplified from the Image clone and digested with BamHI and Xho I. The digested product was then ligated with BamHI and Xho I digested pcDNA6/myc-His C vector. The PCR product includes DNA sequences encoding the leader sequence of uPA for secretion of the expressed uPA (1-135 aa, the ATF domain). The secreted uPA has a myc tag followed by a His tag at its C-terminus (derived from the vector). Two clones were selected for sequence analysis and both were of the correct sequence. One of these clones was used for a large DNA plasmid preparation.

### Expression of uPAR and uPA in transient transfected 239F cells.

Large-scale DNA plasmid preparations were made of both the uPAR and uPA constructs. The DNA plasmids were used for large-scale transient transfection of 293F cells as described below.

293F cells were grown in suspension with 293 Freestyle serum free medium at 37°C with shaking at 125 rpm and 8% CO₂. One day before the transfection, the cells were diluted to a density of 5-6x10⁵/ml. The next day, the cells were diluted to 1x10⁶/ml for transfection.

For each 500 ml of cells, 700 µg of DNA was diluted in 10 ml of Optima MEM medium, and in another tube, 700 µl of 293 Fectin was diluted in 10 ml of Optima MEM medium. 293Fectin dilution was then added into the diluted DNA solution to obtain a total volume of 20 ml. The DNA-lipid mixture was mixed gently and incubated for 10 min at room temperature. The mixture was slowly added into the cell culture and the cells were cultured for additional 10 days.

### Purification of His tagged uPAR and uPA.

The conditioned medium (CM) was collected by centrifugation and filtered with a 0.2 µm filter. The filtered CM was passed through a 20 ml column packed with 1 ml of Ni-NTA (Qiagen, CA) at a flow rate of 1 ml/min.

The beads were washed with 50 ml of wash buffer (300 nM NaCl, 50 mM Tris-HCl, 20 mM imidazole, pH8.0). Bound proteins were eluted 7 ml of elution buffer (300 nM NaCI, 50 mM Tris-HCl, 250 mM imidazole, pH8.0). The eluted proteins were concentrated and buffer exchanged into PBS with a P10 desalting column. An aliquot of each protein preparation was analyzed by SDS-PAGE using a 4-12% gradient SDS PAG (NuPAGE). Coomassie Blue staining revealed single bands of the expected electrophoretic mobility for uPAR (Mr ≈ 45Kd) and uPA (Mr ≈ 20Kd) that made up approximately 90% of the protein in each sample.

### Binding of uPA to uPAR

100 µl of uPAR or BSA at 200 ng/ml were used to coat the wells of an Immulon 4 plate at 4°C overnight. The next day, the wells were washed with TBST (2.5 mM Tris-HCl, 150 mM NaCl, 0.05% Tween 20, pH 8.0) once, then blocked 200 µl of 3 mg/ml BSA in PBS for 1 hr at RT. The wells were washed 3 times with TBST. Serially diluted uPA was added to each wells at triplicate and incubated for 1 hr at RT. The wells were washed with TBST (200 µl/wash) 5 times. 100 µl of anti-myc-HRP (Invitrogen, CA) at a 1:5000 dilution were added to each well and incubated for 1 hr at RT. The wells were washed for 5 times. 100 µl of HRP substrate TMB (KPL, MD) was added to the wells and incubated for 10-15 min. The reaction was stopped with TMB stop solution. The plate was then read at 450 nm. Using Prism software (GraphPad, San Diego CA), the Kd was calculated to be 4.6nM

The library was first double-labeled for scFv expression (Alexa-488-labeled antiV5 tag) and biotinylated-uPAR (antigen) binding. Double positive clones were screened on a FACSAria in rapid screen mode at a rate of 8 x 10⁸ cells / hour. The selected yeast population underwent a second round of selection where antigen binding was performed in the presence of 100 nM uPA (ligand). A population of cells were selected that bound antigen in a manner that was competed by the uAP ligand, indicating that this method should prove capable of identifying scFv antibodies that compete uPA for its ligand binding site on uPAR.

### Example 4: Isolation of an antibody that recognizes the same epitope shared by two or multiple different antigens.

It is often ideal to identify an antibody that recognizes an epitope on antigens from different species, so that the identified antibody can be evaluated in animal models of interest and used in humans. The methods of this invention can also be used to achieve this goal, since each yeast cell is able to display hundreds to thousands copies of the same antibody on its surface.

In this example, a human antigen of interest is labeled in red (no#1 in Figure 3); the rat counterpart of this antigen is labeled in green (no#2 in Figure 3). These two labeled antigens can be added into a suspension of yeast cells displaying a library of antibodies at the same time. After incubation and washing, the yeast cells are subjected to FAC sorting. Yeast cells (such as yeast A in Figure 3) that are double colored are selected, grown, and used for the next round of selection. Multiple rounds of selections can be performed until the majority of cells are double colored. Yeast cells with the brightest red and green are selected for further analysis.

The final antibody selected is able to recognize the antigen from both human and rat. Therefore, this type of antibody can be evaluated in rat for its efficacy before it is tested in humans. Since each yeast cell only expresses one type of antibody, it is highly likely that the selected antibody recognizes the same epitope present in the human and rat antigen. Other antibodies displayed by different yeast cells selected in this way may be able to recognize other epitopes shared by human and rat antigen. Using the method of the invention described in the Example 1, one can further isolate an antibody that recognizes both human and rat antigen, and binds to the epitope of interest. Similarly, this method can be used to isolate antibodies that recognize an epitope that is specific for one antigen member but not for the other in the same protein family, or specific for an antigen from one specie, but from another.

As shown in Figure 3, when a human antigen labeled in red (represented by the molecule with number 1 in it) and the rat counterpart labeled in green (represented by the molecule with number 2 in it) are incubated with a suspension of yeast cells displaying a library of antibodies, yeast cells binding to the human antigen only are red only, whereas yeast cells that bind to rat antigen only is green only. Sorting of yeast cells with red or green antigen bound only allows one to isolate antibody that recognizes the human or rat antigen specifically.

This method is particularly useful for developing antibodies that are specific for a particular member in a large protein family, and are not reactive to other members in the same family. To develop such an antibody to be used as a research reagent, a diagnostic reagent, or a therapeutic, it is often desirable to have an antibody that recognizes only one antigen but not other related molecules that belong to the same protein family and share significant homology to the antigen of interest.

### Example 5: Isolation of an antibody that recognizes a known epitope.

The current invention can also be used to isolate an antibody that recognizes a known epitope. Many antigens from different species are highly homologous to each other. Alignment of antigens from different species allows one to locate epitopes that are different among species. For example, a murine antibody recognizes a human antigen, but does not recognize the mouse counterpart of this antigen. Aligning the human and mouse sequences, one can identify the different epitopes. One of the different epitopes will be that one recognized by the murine antibody.

If such an epitope is known, this murine eptiope can be used to replace the corresponding region in the human antigen. The resulting human-mouse chimera often retains the original conformation of the antigen. The difference between the human and human-mouse chimera is the epitope recognized by the murine antibody. For selecting a fully human antibody that recognizes this epitope, the human antigen is labeled in red (represented by the molecule in complete black), and the human-mouse chimera is labeled in green (represented by the molecule in black with a small circle in white). The labeled antigens can then be used for selection. Yeast cells displaying antibodies that recognize epitopes shared by the human and human-mouse chimera are dual labeled (yeast cell C in Figure 4)

Yeast cells displaying antibodies that recognize the epitope presented by the human antigen, but not by the human-mouse chimera will be red only (yeast cell A). This epitope is the one replaced by mouse sequences in the human-mouse antigen, and is the epitope recognized by the murine antibody. The selected yeast cells in red only, therefore, display antibodies that are able to bind the same epitope recognized by the mouse antibody. Yeast cells displaying an antibody that recognizes the epitope presented by the human-mouse chimera, but not the human antigen will be in green. The displayed antibody therefore recognizes the mouse epitope (yeast cell D).

### Example 6: Isolation and identification of antibodies that recognize disease-specific antigens.

Sample preparation: A biological sample (a tissue, cells, a fluid) is obtained from a patient with a disease of interest. An equivalent sample (the same tissue, cell type and/or fluid) is also obtained from a healthy person, or from healthy/normal regions of the same tissue, cell type of the same patient. The patient and the healthy person are preferably of the same gender, the same ethnic group (or mix of ethnic groups), the same age (or approximately the same age). The patient and the healthy person are preferably also similar in other respects, for example, of similar weight, of similar family history, of similar health history (except for the ongoing disease of the patient). The purpose of the similarities is to maximize the probability that any differences in antigens from the patient and the healthy person are related to the disease of the patient and not to other factors. Hence, the closer the patient and the healthy person match in terms of any factors that are known to, or are likely to, correlate with the antigen composition of the biological samples obtained (except for the ongoing disease of the patient), the better the patient and healthy person are suited for the preparation of antibodies using this embodiment of the invention.

A biological sample may be obtained from the blood, cerebrospinal fluid, the skin, a muscle (skeletal, heart or organ), connective tissue, liver, a tumor, a diseased tissue, kidney, pancreas, gastro-intestinal tissue, esophagus, peripheral nervous system, central nervous system, spinal cord, stomach, spleen, a gland, a lymph node, or any other tissue, organ, or fluid. A biological sample may also be obtained by taking cells, for example, muscle cells, nerve cells, fibroblasts, stem cells, glial cells, cells of the immune system, epithelial cells, diseased cells, endothelial cells, or any other cell type.

A biological sample may be processed to enrich it in certain antigens, for example, a sample may be processed to enrich it in proteins, glycoproteins, phospho-proteins, intracellular proteins, membrane-bound proteins, lipids, glycolipids, peptides, glycopeptides, polynucleotides, oligonucleotides, or any other class of molecules, or by enriching the sample in molecules of one or more of these classes that fall into a certain range or ranges of size (*e.g.,* 1 to 5 kDa, 2 to 8 kDa, 5 to 10 kDa, 10 to 15 kDa, 15 to 20 kDa, 20 to 30 kDa, larger than 30 kDa), pI value (*e.g.,* pH 2, 3, 4, 5, 6, 7, 8, 9, 10, 11 or 12), association with a certain cell type (*e.g.,* nerve cell, fibroblast, muscle cell, fat cell), organelle (*e.g.,* nucleus, endoplasmatic reticulum, Golgi, mitochondria, liposomes, cell membrane, extracellular matrix). A biological sample may be processed using techniques known by those of skill in the art to enrich such a sample as desired.

For example, proteins can be prepared from tissues or cells by lysis of the tissues or cells with lysis buffer, such RIPA (150 mM NaCl, 10 mM Tris, pH 7.2, 0.1% SDS, 1.0% Triton X-100, 1% Deoxycholate, 5 mM EDTA). Lysates can also be passed through a 22 g needle to aid in solubilization. Insoluble materials can be removed by centrifugation. Soluble proteins are saved. If it is not desirable to denature proteins, a non-denaturing solubilization buffer, such as 150 mM NaCl, 20 mM Tris, pH 7.5, 1 % NP40, 5 mM EDTA can be used. Lysates can be homogenized or passed through a needle several times to ensure adequate solubilization. Common abundant proteins in these prepared samples can be removed by affinity depletion; the remaining proteins can be used for labeling and binding to antibody libraries.

Similarly, biofluids such as plasma and serum from a patient and a healthy person can be used to isolate antibodies that are specific to antigens present in the biofluids from the patient but not, or significantly less, in that from the healthy person. The common and highly abundant proteins in plasma, such as albumin, immunoglobulin, transferrin, fibrinogen, can be removed by affinity column, such as ProteomeLab IgY partitioning. The remaining enriched low abundant proteins can be labeled and used in binding to antibodies libraries.

Labeling of antigens: Antigens from a patient and a healthy person, or two different sources, may be labeled in any way that facilitates distinguishing the antigens from each, preferably by means that facilitate FACS. For example, proteins from a patient are labeled with a dye that emits a color, such as red, that is dateable by means such as FACS; proteins from a healthy person are labeled with a dye that emits a different color, such as green. Protein preparation and labeling are carried out in the same way for all samples to minimize extrinsic variables and maximize the intrinsic difference between the two samples.

Contacting antigens with antibodies: Equal amounts of labeled antigens, for example, proteins, from each sample are mixed with an antibody library with antibodies displayed at the surface of a support, such as yeast cells, E. coli, phage, or beads. The exact amount of proteins to be used can be varied and determined empirically based on the binding affinity of the samples to the antibody library, and the signal/noise ratio. The total amounts of the labeled proteins to be used are in certain ranges, such as 1 µg/ml-10 mg/ml, depending on the complexity of antigen samples. The amount can be adjusted so that significant binding of antigens from the first sample can be readily detected with FAC and the two labels are clearly separable. Before mixing the labeled proteins with an antibody library, the antibodies and their support can be blocked with reagents that non-specifically bind to binding sites on the antibodies and their supports, such blocking step may reduce non-specific bindings of the labeled proteins to the displayed antibodies and their support. Blocking reagents can be albumins, milks, and any other types of blocking materials.

After incubation the labeled proteins with the antibody library for a period of time at room temperature or on ice, or any desirable temperatures, unbound proteins are removed by washing the cells (supports) with wash buffer for a few times. To minimize non-specific binding, mild detergent, such as low concentrations of Triton X-100, NP-40 can be included in the wash buffer. The washed cells (supports) are then subjected to screening, such as FAC sorting.

Identifying antibodies of interest: Antibodies that are able to bind proteins presented in both samples in similar concentrations will emit the color used for each sample, for example, red and green. These antibodies recognize proteins common to both samples. Antibodies that are able to bind proteins presented in the sample from patient, but not, or significantly less, in that from the healthy person, only carry the label, and thus emit the color, used for the sample from the patient, for example, red. These antibodies are therefore specific for antigens presented in the sample from the patient, but not in the sample from the healthy person. The cells or supports carrying antibodies with labeled antigens bound thereto, are sorted to separate cells or supports that bind antigens from the patient from the remaining cells or supports. Preferably, FACS sorting is carried out for this purpose.

Further processing of antibodies: The isolated antibodies can be expressed in soluble forms, such as soluble scFv, Fab, F(ab')2 or full IgG and characterized further using standard techniques, such as ELISA, Western blotting, or immunohistochemistry staining, to determine their binding specificity to antigens presented in samples from the patient and the healthy person, and other biological samples as additional controls, if desired.

Antibodies that specifically recognize antigens present in the patient but not, or significantly less, in the healthy person can be further developed into diagnostics for detecting diseases-specific antigens. Such antibodies can also be used to identify the antigens recognized by them with various techniques, such as immunoprecipation and affinity chromatography. The identified antigens can be further characterized and can be used as biomarkers or targets for drug development. The antibodies can also be used in therapy to target therapeutics (including the antibody itself) to diseased tissue.

Similarly, other types of molecules, such as carbohydrates, lipids, or nucleotides can be prepared with the techniques known to those skilled in the arts. The prepared antigens can similarly be labeled and used for isolation and identification of antibodies that recognize disease-specific antigens.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein, will become apparent to those skilled in the art from the foregoing description. Such modifications are intended to fall within the scope of the appended claims. All cited publications, patents, and patent applications are herein incorporated by reference in their entirety.

## Claims

1. A method for obtaining an antibody that specifically recognizes an antigen in a first sample but not in a second sample, said method comprising:
(a) labeling antigens obtained from a first sample and antigens obtained from a second sample, wherein the labeling of antigens from the first and second sample facilitates distinguishing those antigens;
(b) exposing a mixture of antibodies to the labeled antigens from the first and second sample;
(c) selecting antibodies that are identified by detecting the label specific for antigens from said first sample and by not detecting the label specific for antigens from said second sample;
wherein said mixture of antibodies is displayed on a support to facilitate identifying antibody molecules capable of binding antigens presented in the first sample but not presented in the second sample.

2. The method according to claim 1, wherein said first sample is a biological sample from a patient with a disease of interest and said second sample is an equivalent sample from a healthy person.

3. The method according to claim 2, wherein said first sample is a tissue, cells, or a fluid and said second sample is from a healthy person of the same gender and ethnic group as said patient.

4. The method according to claim 2, wherein said first sample is diseased tissue, cells, or a fluid and said second sample is from healthy regions of the same tissue or cell type of said patient.

5. The method according to claim 1, wherein said first sample is from a diseased tissue, cell, or biofluid and said second sample is from a healthy tissue, cell or biofluid.

6. The method according to claims 4 or 5, wherein said first sample is a diseased tissue, cell or biofluid selected from a disease of the group consisting of cancer, diabetes, Alzheimer's, obesity, arthritis, stroke, multiple sclerosis, heart disease, Parkinson's, autoimmune diseases, infectious diseases.

7. The method according to claim 4, wherein said antigens are selected from the group consisting of a protein, a peptides, a glycoprotein, a carbohydrate, a lipid, and a polynucleotide.

8. The method according to claim 1, wherein said mixture of antibodies is a library of antibodies derived from any source.

9. The method according to claim 8, wherein said library of antibodies comprises synthetic variable regions with randomized amino acid sequences in the CDRs and human heavy and light chain germline frameworks.

10. An engineered library of polynucleotides comprising a framework of a single human germline VH gene and a framework of a single human germline VL gene, and further comprising CDRH3 from an antibody of interest and a collection of randomized CDRH1, CDRH2, CDRL1, CDRL2 and CDRL3.


SEQUENCE LISTING

<110> Xiaomin Fan
      Xiaoming Chen

<120> Methods for the identification and the
  isolation of epitope specific antibodies

<130> C14421EP

<150> 60/802,085
<151> 2006-05-18

<160> 55

<170> FastSEQ for Windows Version 4.0

<210> 1
<211> 23
<212> DNA
<213> mouse

<400> 1
ctcaccggtc gcgttcctga aac                                              23

<210> 2
<211> 89
<212> DNA
<213> mouse

<400> 2
cgggtttaaa ccaggcgcgg ccgctgtcgc tagcgcggcc ggatccaagt agaattccct 60
taattgaaaa tgtatgaagt agaattagc                                        89

<210> 3
<211> 40
<212> DNA
<213> mouse

<400> 3
ggtagcggcc gcccaggaac tgacaactat atgcgagcaa                            40

<210> 4
<211> 42
<212> DNA
<213> mouse

<400> 4
cgggtttaaa ctcaaaaaca tactgtgtgt ttatggggct gc                         42

<210> 5
<211> 23
<212> PRT

<213> mouse

<400> 5
Asp Ile Gln Met Thr Gln Ser Pro Ser Ser Leu Ser Ala Ser Val Gly
1               5                   10                  15
Asp Arg Val Thr Ile Thr Cys
            20

<210> 6
<211> 11
<212> PRT
<213> mouse

<400> 6
Arg Ala Ser Gln Xaa Xaa Xaa Xaa Xaa Leu Asn
1               5                   10

<210> 7
<211> 15
<212> PRT
<213> mouse

<400> 7
Trp Tyr Gln Gln Lys Pro Gly Lys Ala Pro Lys Leu Leu Ile Tyr
1               5                   10                  15

<210> 8
<211> 7
<212> PRT
<213> mouse

<400> 8
Xaa Ala Ser Xaa Leu Xaa Ser
1               5

<210> 9
<211> 32
<212> PRT
<213> mouse

<400> 9
Gly Val Pro Ser Arg Phe Ser Gly Ser Gly Ser Gly Thr Asp Phe Thr
1               5                   10                  15
Leu Thr Ile Ser Ser Leu Gln Pro Phe Ala Phe Ala Thr Tyr Tyr Cys
            20                  25                  30

<210> 10
<211> 9
<212> PRT
<213> mouse

<400> 10
Gln Gln Xaa Xaa Xaa Xaa Pro Xaa Thr
1               5

<210> 11
<211> 10
<212> PRT
<213> mouse

<400> 11
Phe Gly Gln Gly Thr Lys Val Glu Ile Lys
1               5                   10

<210> 12
<211> 25
<212> PRT
<213> mouse

<400> 12
Glu Val Gln Leu Leu Glu Ser Gly Gly Gly Leu Val Gln Pro Gly Gly
1               5                   10                  15
Ser Leu Arg Leu Ser Cys Ala Ala Ser
            20                  25

<210> 13
<211> 10
<212> PRT
<213> mouse

<400> 13
Gly Phe Thr Phe Xaa Xaa Xaa Xaa Met Ser
1               5                   10

<210> 14
<211> 14
<212> PRT
<213> mouse

<400> 14
Trp Val Arg Gln Ala Pro Gly Lys Gly Leu Glu Trp Val Xaa
1               5                   10

<210> 15
<211> 17
<212> PRT
<213> mouse

<400> 15
Xaa Ile Xaa Pro Xaa Xaa Gly Xaa Thr Xaa Tyr Ala Asp Ser Val Lys

1               5                   10                  15
Gly

<210> 16
<211> 32
<212> PRT
<213> mouse

<400> 16
Arg Phe Thr Ile Ser Arg Asp Asn Ser Lys Asn Thr Leu Tyr Leu Gln
1               5                   10                  15
Met Asn Ser Leu Arg Ala Glu Asp Thr Ala Val Tyr Tyr Cys Ala Arg
            20                  25                  30

<210> 17
<211> 12
<212> PRT
<213> mouse

<400> 17
Xaa Xaa Xaa Xaa Xaa Xaa Xaa Xaa Xaa Met Asp Tyr
1               5                   10

<210> 18
<211> 11
<212> PRT
<213> mouse

<400> 18
Trp Gly Gln Gly Thr Leu Val Thr Val Ser Ser
1               5                   10

<210> 19
<211> 27
<212> DNA
<213> mouse

<400> 19
gacaccgctg tttactactg cgctcgc                                          27

<210> 20
<211> 23
<212> DNA
<213> mouse

<400> 20
gtaccctgac cccagtagtc cat                                              23

<210> 21
<211> 22

<212> DNA
<213> mouse

<400> 21
aagatgcagt tacttcgctg tt                                                22

<210> 22
<211> 18
<212> DNA
<213> mouse

<400> 22
aacggtgaaa ccggaagc                                                     18

<210> 23
<211> 21
<212> DNA
<213> mouse

<400> 23
gctgcttccg gtttcaccgt t                                                 21

<210> 24
<211> 18
<212> DNA
<213> mouse

<400> 24
ctgacgaacc caggacat                                                     18

<210> 25
<211> 21
<212> DNA
<213> mouse

<400> 25
ggtaaaggtc tggaatgggt t                                                 21

<210> 26
<211> 21
<212> DNA
<213> mouse

<400> 26
acctttaacg gagtcagcgt a                                                 21

<210> 27
<211> 21
<212> DNA
<213> mouse

<400> 27
tacgctgact ccgttaaagg t                                                 21

<210> 28

<211> 24
<212> DNA
<213> mouse

<400> 28
tctagcgcag tagtaaacag cggt                                          24

<210> 29
<211> 43
<212> DNA
<213> mouse

<400> 29
ttcgactact ggggtcaggg taccctggtt accgtttcct ccg                     43

<210> 30
<211> 84
<212> DNA
<213> mouse

<400> 30
ggactgggtc atctggatgt cagaaccacc gccaccggaa ccgccaccac cggagccacc 60
gcctccggag gaaacggtaa ccag                                          84

<210> 31
<211> 21
<212> DNA
<213> mouse

<400> 31
gacatccaga tgacccagtc c                                             21

<210> 32
<211> 21
<212> DNA
<213> mouse

<400> 32
ttgggaagca cggcaggtga t                                             21

<210> 33
<211> 21
<212> DNA
<213> mouse

<400> 33
atcacctgcc gtgcttccca a                                             21

<210> 34
<211> 30
<212> DNA
<213> mouse

<400> 34
tttacccggt ttctgctggt accaattcag                                    30

<210> 35
<211> 24
<212> DNA
<213> mouse

<400> 35
aaagctccga aactgctgat ttac                                              24

<210> 36
<211> 24
<212> DNA
<213> mouse

<400> 36
acgggacgga acaccagaag ccag                                              24

<210> 37
<211> 24
<212> DNA
<213> mouse

<400> 37
acgggacgga acaccagact gcag                                              24

<210> 38
<211> 24
<212> DNA
<213> mouse

<400> 38
acgggacgga acaccagatt ccag                                              24

<210> 39
<211> 18
<212> DNA
<213> mouse

<400> 39
tctggtgttc cgtcccgt                                                     18

<210> 40
<211> 25
<212> DNA
<213> mouse

<400> 40
gttggcagta gtaggtagcg aagtc                                             25

<210> 41
<211> 21
<212> DNA
<213> mouse

<400> 41

gctacctact actgccaaca a                                                21

<210> 42
<211> 21
<212> DNA
<213> mouse

<400> 42
cttggtacct tgaccgaagg t                                                21

<210> 43
<211> 44
<212> DNA
<213> mouse

<400> 43
acccattcca gacctttacc cggagcctga cgaacccagg acat                       44

<210> 44
<211> 51
<212> DNA
<213> mouse

<400> 44
ctgaattggt accagcagaa accgggtaaa gctccgaaac tgctgattta c               51

<210> 45
<211> 60
<212> DNA
<213> mouse

<400> 45
gttagggata ggcttacccc tcgagccctt gatctcaacc ttggtacctt gaccgaaggt 60


<210> 46
<211> 27
<212> DNA
<213> mouse

<400> 46
cggtttgtat tacttcttat tcaaatg                                          27

<210> 47
<211> 20
<212> DNA
<213> mouse

<400> 47
ctccttgcat tgccttcccg                                                  20

<210> 48
<211> 26
<212> DNA
<213> mouse

<400> 48
tacctctata ctttaacgtc aaggag                                          26

<210> 49
<211> 26
<212> DNA
<213> mouse

<400> 49
ggccaaaata gtagtcgttg acaaag                                          26

<210> 50
<211> 25
<212> DNA
<213> mouse

<400> 50
ctgcataacc actttaacta atact                                           25

<210> 51
<211> 24
<212> DNA
<213> mouse

<400> 51
ctgaagcaat aacagaaaat attg                                            24

<210> 52
<211> 37
<212> DNA
<213> mouse

<400> 52
ccggatccgc cgccaccatg ggtcacccgc cgctgct                              37

<210> 53
<211> 30
<212> DNA
<213> mouse

<400> 53
atgaccggtc ccactgcggt actggacatc                                      30

<210> 54
<211> 29
<212> DNA
<213> mouse

<400> 54
cgggatccgc cgccaccatg agagccctg                                       29

<210> 55
<211> 31
<212> DNA

<213> mouse

<400> 55
ccgctcgagc ttttccatct gcgcagtcat g                                    31